# FASCICULE DE BREVET EUROPEEN

(11) **EP 3 762 001 B1**
(45) Date de publication et mention de la délivrance du brevet: **21.08.2024**
(21) Numéro de dépôt: 19713110.5
(22) Date de dépôt: 08.03.2019
(51) Int. Cl.: A61K 35/74, A01N 1/02, C12Q 1/04, A61P 1/00, A61P 1/12

(54) **PROCÉDURE DE COLLECTE DE SELLES ET PROCÉDÉ DE PRÉPARATION D'UN ÉCHANTILLON POUR TRANSPLANTATION DE MICROBIOTE FÉCAL**
STUHLSAMMELVORRICHTUNG UND PROBEZUBEREITUNG ZUR DARMFLORATRANSPLANTATION
STOOL COLLECTING PROCEDURE AND PROCESS OF PREPARATION OF SAMPLE FOR FECAL TRANSPLANTATION

(30) Priorité: 09.03.2018 FR 1852084
(43) Date de publication de la demande: 13.01.2021
(73) Titulaire: Maat Pharma, 69007 Lyon (FR)
(72) Inventeur: SCHWINTNER, Carole, 69007 LYON (FR); LEROUX, Alice, 69007 LYON (FR); MADER, Clémence, 69007 LYON (FR); AFFAGARD, Hervé, 69007 LYON (FR)
(74) Mandataire: Santarelli
(86) Numéro de dépôt international: PCT/FR2019/050522
(87) Numéro de publication internationale: WO 2019/171012

(56) Documents cités:
- WO-A1-2016/170285
- WO-A1-2018/026913
- CHEN JUSTIN: "From Stool Donation to Fecal Microbiota Preparation The Logistics of Fecal Microbiota Preparation Manufacturing", OPENBIOME.ORG, 12 August 2021 (2021-08-12), XP055876907, Retrieved from the Internet <URL:https://static1.squarespace.com/static/50e0c29ae4b0a05702af7e6a/t/6112851ea43dfb2a47768da7/1628603678547/Manufacturing-FMT.pdf> [retrieved on 20220107]
- PARAMSOTHY SUDARSHAN ET AL: "Multidonor intensive faecal microbiota transplantation for active ulcerative colitis: a randomised placebo-controlled trial", LANCET (NORTH AMERICAN EDITION), vol. 389, no. 10075, 14 February 2017 (2017-02-14), pages 1218 - 1228, XP002785429
- JACOB V ET AL: "Single Delivery of High-diversity Fecal Microbiota Preparation by Colonoscopy is Safe and Effective in Increasing Microbial Diversity in Active Ulcerative Colitis", HHS PUBLIC ACCESS AUTHOR MANUSCRIPT, vol. 23, no. 6, 1 June 2017 (2017-06-01), pages 1 - 18, XP002791475, DOI: 10.1097/MIB.0000000000001132

## Description

La présente invention se rapporte à une procédure de collecte de selles de plusieurs donneurs et un procédé de préparation d'un échantillon de microbiote fécal.

L'invention se rapporte également à l'utilisation dudit échantillon dans la transplantation de microbiote fécal (TMF, aussi appelé transfert de microbiote fécal), de préférence pour traiter les dysbioses intestinales.

Le microbiote intestinal humain, couramment appelé « flore intestinale », est l'ensemble des micro-organismes (bactéries, levures et champignons) qui se trouvent dans le système gastro-intestinal humain (estomac, intestin et côlon). La diversité bactérienne est estimée à l'heure actuelle à environ 10³ espèces bactériennes composant le microbiote intestinal dominant d'un individu adulte, avec une abondance de 10¹⁴ bactéries, représentant un métagénome bactérien de 200 000 à 800 000 gènes chez chaque individu, soit 10 à 50 fois le nombre de gènes du génome humain. Stérile *in utero,* l'intestin se colonise dès les premiers jours de vie jusqu'à évoluer vers un microbiote individuel unique. Chaque personne possède des bactéries relativement proches en termes d'espèces, mais la composition exacte de son microbiote (espèces, proportions) est pour une large part (plus de 2/3 des espèces) spécifique de l'hôte. Ainsi, le microbiote intestinal humain est un écosystème très diversifié, complexe et spécifique de chaque individu.

Le maintien d'une grande diversité du microbiote favorise sa stabilité. Cependant, certaines pathologies ou traitements déséquilibrent le microbiote : les antibiotiques par exemple, ainsi que les maladies à composante inflammatoire, telles que les maladies inflammatoires chroniques de l'intestin (MICI), peuvent limiter la diversité du microbiote dans l'intestin. Les traitements antibiotiques (ou antibiothérapie), notamment, résultent en une altération du microbiote, ce qui peut favoriser la prolifération d'organismes pathogènes comme *Clostridium difficile.*

Un certain nombre de pathologies sont associées à une dysbiose intestinale, par exemple la maladie du greffon contre l'hôte (Graft-Versus-Host Disease - GvHD). La greffe ou transplantation allogénique de cellules souches hématopoïétiques (allo-HSCT) est un traitement efficace contre les malignités hématopoïétiques et les troubles hématopoïétiques héréditaires, et elle est considérée comme l'immunothérapie tumorale la plus efficace à ce jour [Sung, A.D. and Chao, N.J. (2013), Concise Review: Acute Graft-Versus-Host Disease: Immunobiology, Prevention, and Treatment, Stem Cells Transl. Med. 2: 25-32]. Cependant, les lymphocytes T dérivés de cellules souches transplantées peuvent attaquer les tissus récepteurs hôtes entraînant la GvHD, complication majeure de l'allo-HSCT associée à une mortalité significative (15 à 25% des décès après une allo-HSCT). Les patients subissant une allo-HSCT peuvent être simultanément exposés à une chimiothérapie cytotoxique, à une irradiation corporelle totale, à des immunosuppresseurs et à des antibiotiques à large spectre pouvant causer des altérations importantes du microbiote intestinal. En effet, un enrichissement important des Enterococcaceae, ainsi qu'une augmentation de Lactobacillales et une diminution de Clostridiales, est souvent observé chez les patients après une allo-HSCT. En conséquence, des bactéries dominantes communément rencontrées telles que *Enterococcus* résistant à la vancomycine, *Streptococcus* du groupe des viridans et diverses protéobactéries, peuvent entrer dans le sang et provoquer une septicémie. Il est intéressant de noter que ce changement est particulièrement important chez les patients qui développent ensuite une maladie réfractaire du greffon contre l'hôte (GvHD) [Holler et al. (2014), Biol Blood Marrow Transplant. 20(5): 640-645, et Peled, J. (2017) 59th Annual Meeting of the American Society of Hematology, Atlanta, USA, Dec 9-11].

Afin de rétablir le microbiote intestinal et ainsi rétablir l'homéostasie (i.e. la symbiose), la transplantation de microbiote fécal (TMF) est envisagée et testée. Elle consiste en l'introduction des selles d'un donneur sain dans le tube digestif d'un patient receveur, afin de rééquilibrer le microbiote intestinal altéré de l'hôte. Cette transplantation de microbiote fécal peut être allogénique (c'est-à-dire d'un individu donneur sain vers un patient) ou autologue (c'est-à-dire d'un individu vers lui-même). Les résultats obtenus sur les infections de type *Clostridium difficile* sont encourageants, et certains patients ont été traités avec succès (Tauxe et al, Lab Medicine, Winter 2015, volume 46, Numéro 1). Des études récentes ont aussi démontré que les patients GvHD traités avec TMF ont eu une amélioration des symptômes gastro-intestinaux et ont eu une réduction ou disparition des diarrhées, associée à une reconstruction du microbiote [Kakihana et al. (2017) Transplantation 128 : 2083-2089, et Spindelboeck et al. (2017) Hematologica 102 : e210].

Dans le cas de la transplantation allogénique, il est important que l'échantillon transplanté ait un profil acceptable en termes de viabilité et de diversité des bactéries, car la suspension de microbiote dans son diluant représente le principe actif (substance active) du médicament. Les méthodes de transplantation actuelles sont souvent empiriques et ne prennent pas de précautions particulières pour assurer la diversité des bactéries présentes dans les échantillons de microbiote fécal utilisés, ni pour préserver au mieux la viabilité des bactéries anaérobies, composants majoritaires du microbiote intestinal.

La demande de brevet américaine US 2017/239303 décrit des compositions pour la restauration du microbiote intestinal, ainsi que leurs procédés de fabrication et leur administration. Ce document divulgue que les compositions comprenant des échantillons ont un indice de diversité de Shannon d'environ 0.4-2.5 où les calculs sont effectués au niveau de la famille. Il est de l'ordre de 1-8 selon le niveau de taxonomie (phyla, espèces etc.) en fonction duquel la diversité est calculée.

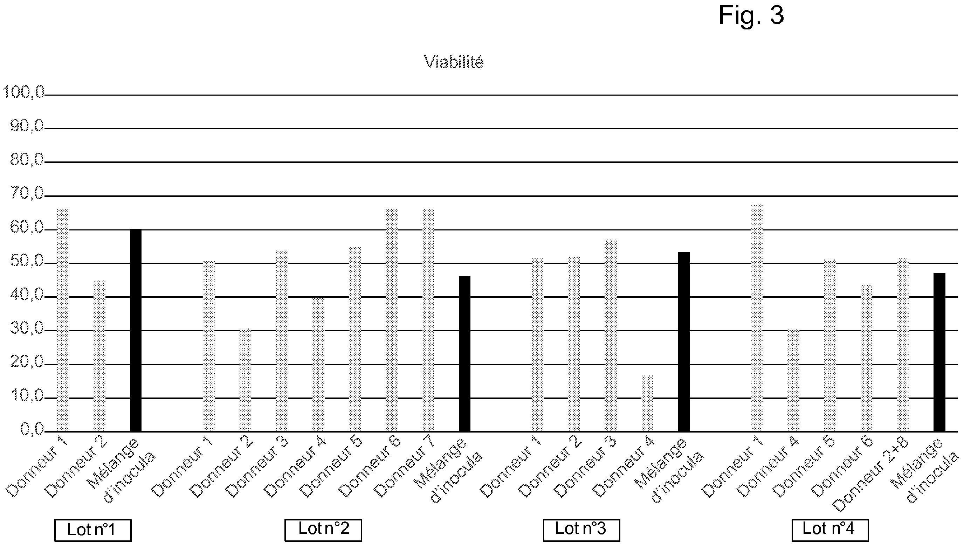

La publication Paramsothy et al. [Paramsothy S., et al. (2017), Multidonor intensive faecal microbiota transplantation for active ulcerative colitis: a randomised placebo-controlled trial, Lancet, Mar 25; 389(10075): 1218-1228] décrit la préparation des compositions de microbiote fécal dans le traitement de la colite ulcéreuse active. Les compositions sont produites par le mélange et l'homogénéisation des selles de 3-7 donneurs, puis une solution aqueuse saline est ajoutée au mélange obtenu (voir page 122, colonne de gauche, « Procédures »). La Figure 3 de la publication de Paramsothy et al. (panneau de droite en haut) montre la diversité phylogénétique des compositions. Une variance importante existe toujours entre les lots produits (indiqué par « Donor batches » dans la Figure), même après avoir regroupé les échantillons de selles des donneurs. Cette variance semble aussi importante que celle entre les donneurs individuels (indiqué par « Individual Donors» dans la Figure). Ainsi, une hétérogénéité importante existe entre les échantillons destinés à traiter les patients. Une telle hétérogénéité n'est pas souhaitable pour une composition pharmaceutique. En effet, pour s'assurer que le patient répond de la même manière à chaque dose (ou échantillon) de microbiote fécal qu'il reçoit, il est nécessaire que les doses soient aussi homogènes que possible, et que les lots soient homogènes également.

Il existe donc un besoin de fournir un procédé de transplantation de microbiote fécal qui soit sécurisé, reproductible, efficace et facile à mettre en oeuvre, notamment à l'échelle industrielle. En outre, il existe un besoin pour un procédé de transplantation de microbiote fécal dans lequel la diversité bactérienne des produits transplantés est la plus élevée possible et dans lequel une homogénéité existe entre les différentes doses (échantillons) de produits transplantés à la même personne. Ainsi, il est nécessaire qu'il existe de l'homogénéité entre les différentes doses issues du même lot de microbiote fécal (homogénéité intra-lot) et entre les différents lots produits (homogénéité inter-lots). La viabilité des bactéries doit être conservée autant que possible au cours du procédé et pendant le stockage.

Il existe un besoin de fournir des échantillons de microbiote fécal ayant une diversité et une viabilité bactérienne optimales, à administrer pour le traitement et la prévention de la dysbiose intestinale bactérienne (iatrogène ou non-iatrogène) et/ou des pathologies associées. Il existe un besoin de fournir un procédé de préparation de tels échantillons. Les pathologies concernées peuvent être la maladie réfractaire du greffon contre l'hôte (GvHD), l'infection à *Clostridium difficile,* la rectocolite hémorragique, la maladie intestinale inflammatoire, le syndrome du côlon irritable, la maladie de Crohn, le diabète de type II, les allergies alimentaires, le cancer, y compris la leucémie, l'obésité et l'obésité morbide. D'autres pathologies associées à la dysbiose comprennent l'autisme, la sclérose, la diarrhée du voyageur, la dysbiose liée aux soins intensifs à l'hôpital, la maladie de Parkinson, la maladie d'Alzheimer, la schizophrénie, la dysbiose intestinale associée à une chimiothérapie anticancéreuse ou à une immunothérapie, la dysbiose liée aux maladies alcooliques et non-alcooliques du foie.

Il est nécessaire de fournir des échantillons de microbiote fécal à utiliser dans le traitement ou la prévention de la dysbiose intestinale iatrogène et/ou des pathologies et complications associées comprenant, mais sans s'y limiter, l'inflammation intestinale et la diarrhée.

La présente invention répond aux besoins décrits ci-dessus.

Par conséquent, un objet de l'invention est de fournir des échantillons de microbiote fécal, ayant une diversité optimale et une viabilité bactérienne suffisante, pour leur utilisation dans le TMF (Transfert de Microbiote Fécal), et qui peuvent être facilement produits d'une manière fiable et reproductible.

L'invention concerne un procédé de préparation d'un mélange homogène de microbiote fécal provenant d'au moins deux donneurs présélectionnés comprenant les étapes suivantes :
a. le prélèvement d'au moins un échantillon de microbiote fécal desdits donneurs présélectionnés,
b. dans un délai inférieur à 5 minutes suivant le prélèvement, le placement de l'échantillon obtenu en a) dans un dispositif de collecte étanche à l'oxygène,
c. le contrôle qualitatif des échantillons prélevés et l'exclusion des échantillons ne répondant pas aux critères qualitatifs,
d. l'ajout à chacun des échantillons retenus après l'étape de contrôle c) d'une solution aqueuse saline comprenant au moins un agent cryoprotecteur et/ou un agent de charge,
e. la filtration des échantillons obtenus à l'issue de l'étape d) pour former une série d'inocula,
f. le regroupement desdits inocula pour former un mélange d'inocula,
g. l'homogénéisation dudit mélange obtenu à l'étape f), notamment par agitation manuelle ou à l'aide d'un dispositif d'agitation, les étapes b) et d) à g) étant réalisées en anaérobiose.

Selon un mode de réalisation de l'invention, les donneurs sont présélectionnés selon les critères de présélection suivants :
i. ayant entre 18 et 60 ans,
ii. ayant un Indice de Masse Corporel (IMC) entre 18 et 30,
iii. absence d'antécédents personnels de maladies infectieuses graves, telles que le SIDA, l'hépatite etc., de troubles métaboliques et neurologiques, ou de dépression,
iv. absence de prise récente de médicaments pouvant détériorer la composition du microbiote intestinal,
v. absence d'apparition récente de symptômes associés à une maladie gastro intestinale, tels que fièvre, diarrhée, nausée, vomissement, douleur abdominale, jaunisse, absence d'historique de maladies infectieuses graves, notamment le SIDA, l'hépatite etc .
vi. absence de voyages récents dans des pays tropicaux,
vii. absence de comportement sexuel à risque,
viii. absence de blessure, piercing et/ou tatouage récent(s) (typiquement, dans les dernier trois mois),
ix. absence de fatigue chronique récente (typiquement, dans les dernier trois mois),
x. absence de réaction allergique récente (typiquement, dans les dernier trois mois),
xi. optionnellement, ayant un régime alimentaire divers.

Selon un mode de réalisation de l'invention, les critères qualitatifs des échantillons de l'étape c) comprennent :
- consistance de l'échantillon entre 1 et 6 selon l'échelle de Bristol,
- absence de sang et d'urine dans l'échantillon.

Selon un mode de réalisation de l'invention, les critères qualitatifs des échantillons de l'étape c) comprennent :
- absence des bactéries suivantes : *Campylobacter, Clostridium difficile (toxin A*/*B), Salmonella Yersinia enterocolitica, Vibrio sp., Shigo-like toxin-producing E.coli (STEC) stxl*/*stx2,* des bactéries multi résistantes, bactéries productrices de Bêta-lactamase à spectre étendu (BLSE) - Entérocoques résistant à la vancomycine et aux glycopeptides (ERV, GRE), *Listeria monocytogenes,* et les *bactéries résistantes aux carbapénèmes,*
- absence des parasites suivants : *Cryptosporidium parvum,* Cyclospora sp., *Entamoeba histolytica, Giardia lamblia, Blastocystis hominis, Helminths, Strongyloides stercoralis, Isospora sp., Microsporidies et Dientamoeba fragilis,*
- absence des virus suivants : Adenovirus F40/41, Astrovirus, Norovirus, Rotavirus A, Sapovirus et Picornavirus (Aichi Virus et enterovirus),
- absence des bactéries suivantes : *E.coli enteroaggregative* (EAEC), E*.coli enteropathogenic* (EPEC), *E.coli enterotoxigenic* (ETEC) It/st, *Shigella*/*Enteroinvasive E.coli* (EIEC) et *Plesiomonas shigelloides.*

Selon un mode de réalisation de l'invention, l'au moins un agent cryoprotecteur et/ou agent de charge de l'étape d) est un polyol, un di-, tri- ou polysaccaride ou leur mélange et un agent de charge.

Selon un mode de réalisation de l'invention, la solution aqueuse saline de l'étape d) comprend maltodextrine et tréhalose.

Selon un mode de réalisation de l'invention, la filtration à l'étape e) est réalisée avec un filtre comprenant des pores de diamètre inférieur ou égal à 0,5 mm, de préférence inférieur ou égal à 265 µm.

Selon un mode de réalisation de l'invention, le temps entre la collecte de l'échantillon et la fin de l'étape g) est moins de 76 heures.

Selon un mode de réalisation de l'invention, l'étape g) d'homogénéisation est réalisée à une température entre 2°C et 25°C, de préférence entre environ 2 et 6°C, plus préférentiellement à environ 4°C.

Selon un mode de réalisation de l'invention, le procédé comprend l'étape de transfert suivante :
h. le transfert du mélange homogénéisé obtenu à l'étape g) :
i. dans des poches pour stockage à une température d'environ -50°C à -80°C, de préférence, à -80°C, ou pour stockage à une température entre environ 2 à 6 °C pour utilisation du mélange sous 16 heures environ, ou pour stockage à une température entre 10 à 25 °C pour utilisation du mélange sous 4 heures environ,
ii. dans un dispositif de lyophilisation pour lyophilisation.

Selon un mode de réalisation de l'invention, le mélange homogène de microbiote fécal provient d'au moins quatre donneurs, de préférence d'au moins cinq donneurs.

Selon un autre aspect, l'invention concerne l'utilisation du mélange homogène de microbiote fécal susceptible d'être obtenu selon le procédé de l'invention pour son utilisation dans le traitement des dysbioses intestinales et/ou des maladies associées aux dysbioses intestinales par transplantation de microbiote fécal (TMF) allogénique.

Selon un mode de réalisation de l'invention, le mélange homogène de microbiote fécal provient d'au moins quatre donneurs et contient les genres bactériens producteurs de butyrate suivants : *Blautia, Faecalibacterium, Alistipes, Eubacterium, Bifidobacterium, Ruminococcus, Clostridium, Coprococcus, Odoribacter, Roseburia, Holdemanella, Anaerostipes, Oscillibacter, Subdoligranulum* et *Butyrivibrio.*

Selon un mode de réalisation de l'invention, ledit mélange homogène de microbiote fécal a une diversité élevée, ayant un inverse de l'indice de Simpson supérieur à 15, de préférence, supérieur à 20.

L'invention concerne l'utilisation du mélange homogène de microbiote fécal susceptible d'être obtenu selon le procédé de l'invention dans le traitement des dysbioses intestinales.

L'invention concerne l'utilisation du mélange homogène de microbiote fécal susceptible d'être obtenu selon le procédé de l'invention dans le traitement de la maladie du greffon contre l'hôte (GvHD).

L'invention concerne l'utilisation du mélange homogène de microbiote fécal susceptible d'être obtenu selon le procédé de l'invention dans le traitement de la dysbiose intestinale iatrogène et/ou des pathologies et complications associées comprenant l'inflammation intestinale et la diarrhée.

L'invention concerne l'utilisation du mélange homogène de microbiote fécal susceptible d'être obtenu selon le procédé de l'invention dans le traitement de l'infection à *Clostridium difficile* et la diarrhée associée (CDI), la maladie intestinale inflammatoire (IBD), le syndrome du côlon irritable (IBS), la constipation idiopathique, la maladie coeliaque, la maladie de Crohn, l'obésité et l'obésité morbide, l'autisme, la sclérose en plaques, la diarrhée du voyageur, la maladie de Parkinson, le diabète de type II, les allergies alimentaires, le cancer, la maladie d'Alzheimer, la schizophrénie et les troubles bipolaires, la dysbiose intestinale associée à une chimiothérapie anticancéreuse ou à une immunothérapie et la maladie alcoolique et non-alcoolique du foie.

### Description des Figures

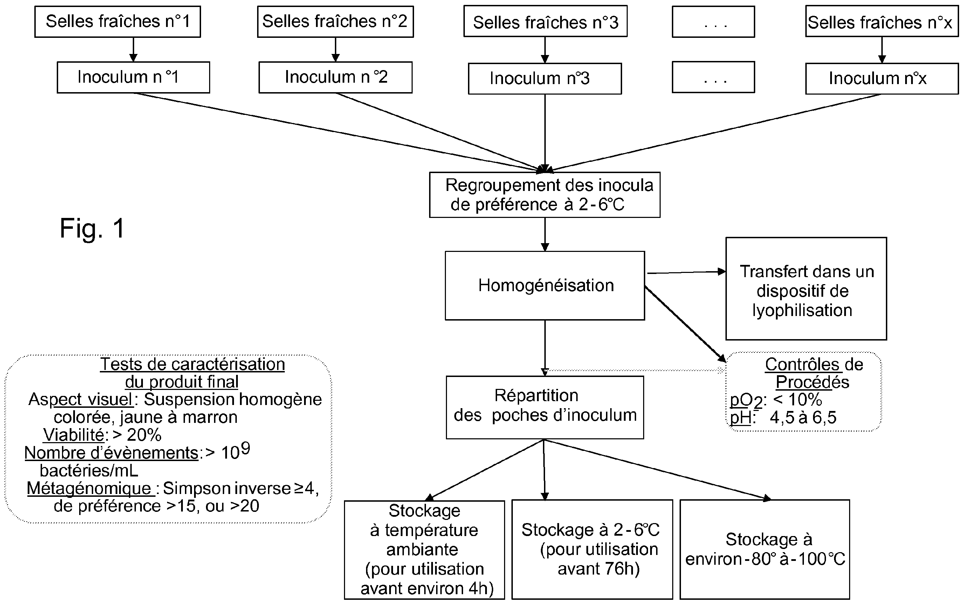

La Figure 1 est une représentation schématique du procédé de préparation d'un mélange homogène de microbiote fécal à partir des échantillons de microbiote fécal de plusieurs donneurs pour utilisation en TMF allogénique.
La Figure 2 est un histogramme représentant le pourcentage de viabilité du microbiote de l'inoculum (inoculum 1, 4, 5, 6 et 2 + 8) et du mélange d'inoculum (substance active) dans des poches 1 à 15 d'inoculum pour le stockage.
La Figure 3 est un histogramme représentant le pourcentage de viabilité du microbiote des inocula individuels et des mélanges d'inocula (inocula regroupés) pour quatre lots de produit. Le lot N° 4 est le même lot que celui utilisé pour l'Exemple 1 (et illustré dans la Figure 2).
Les Figures 4a et 4b montrent la richesse et la diversité bactérienne des inocula.

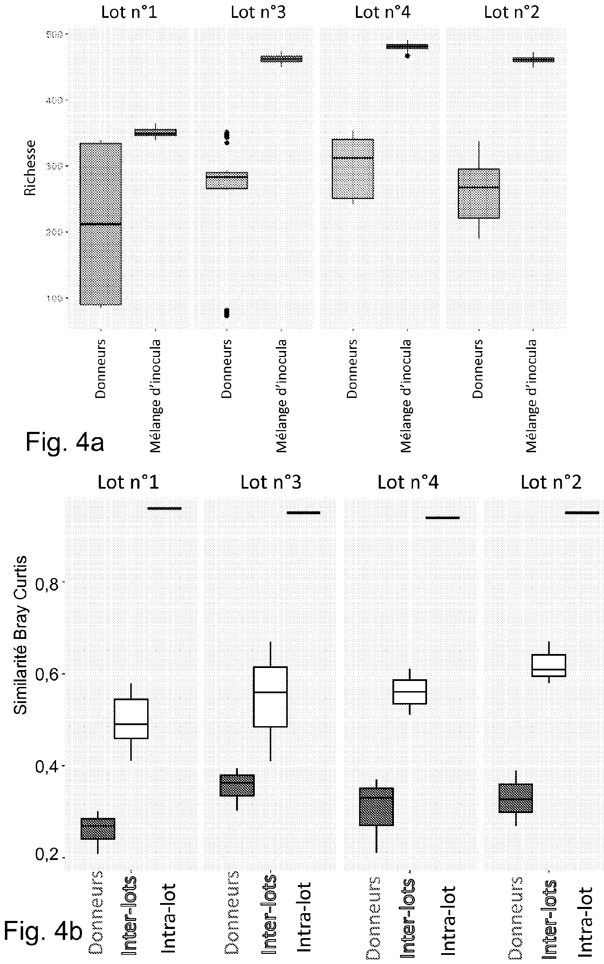

Figure 4a : La richesse (nombre d'espèces bactériennes) mesurée pour les inocula individuels et aussi pour le mélange d'inocula pour des lots, lot N° 1 à lot N°4. Les unités taxonomiques opérationnelles (en anglais, operational taxonomie units (OTUs)) ont été évaluées en ARN ribosomique 16S (ARNr 16S). Pour des donneurs individuels, la richesse est entre 100 et 350 espèces.
Figure 4b : La similarité Bray Curtis des inocula individuels par lot (« Donneurs »), des inocula comparés entre lots (« Inter-lots ») et des inocula regroupés dans les poches dans le même lot (« Intra-lot »).

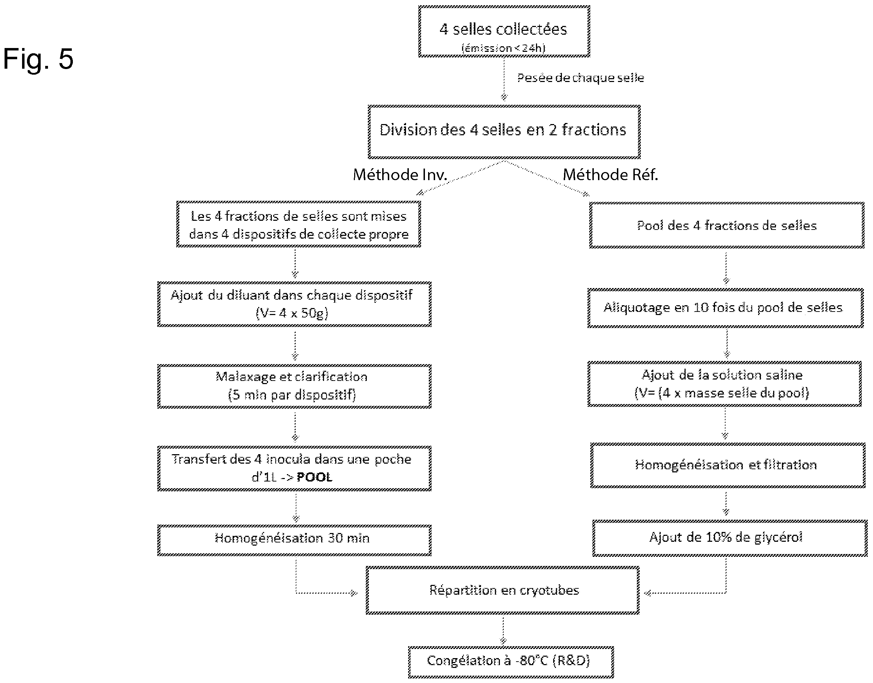

La Figure 5 est une représentation schématique des expériences comparatives de l'Exemple 3, dans lesquelles les mêmes selles ont été utilisées pour la réalisation des deux fabrications de lots d'échantillons, une fabrication selon un mode de réalisation de l'invention (« Méthode Inv. »), identique à la méthode décrite pour l'Exemple 3, et une fabrication selon la méthode décrite dans Paramsothy et al. [Paramsothy S., et al. (2017), Multidonor intensive faecal microbiota transplantation for active ulcerative colitis: a randomised placebo-controlled trial, Lancet, Mar 25; 389(10075): 1218-1228], ou « Méthode Réf ».

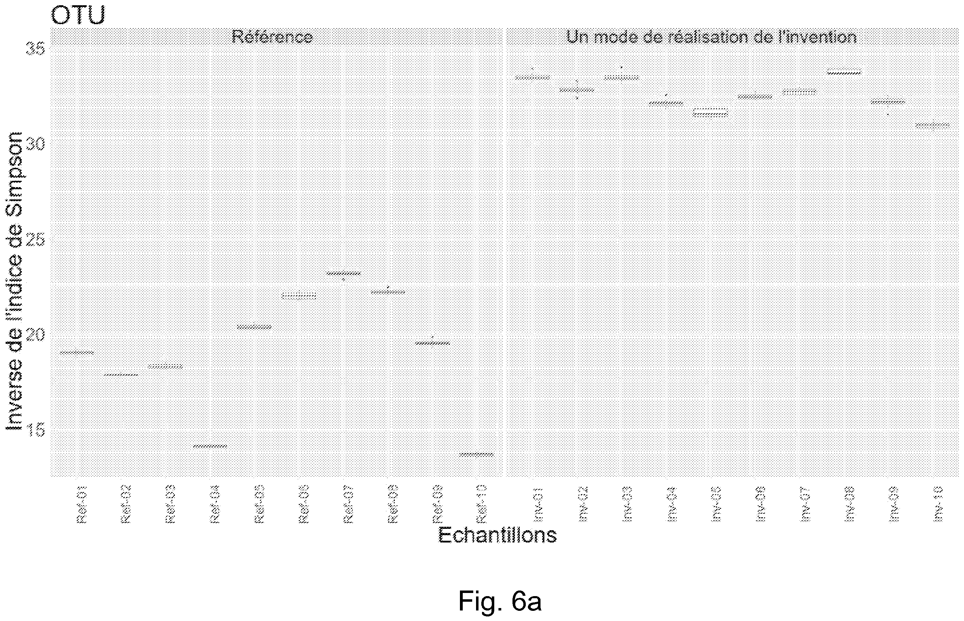

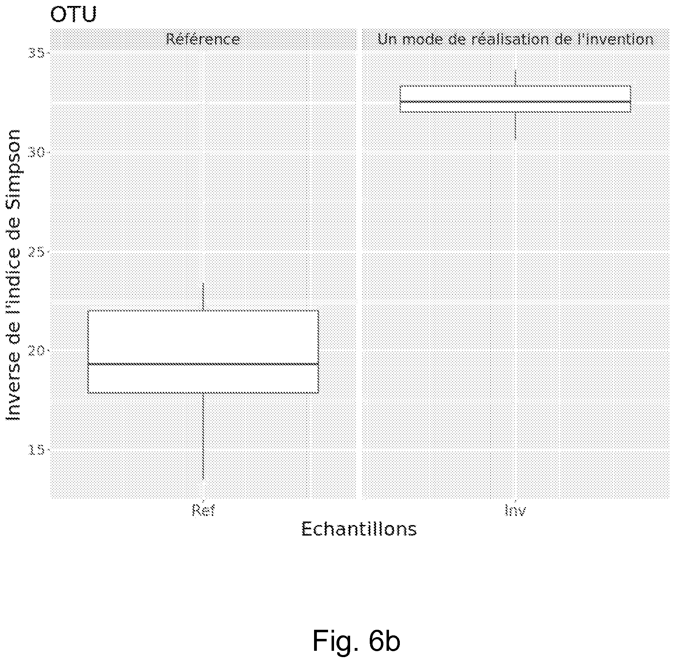

La Figure 6 représente la diversité mesurée selon l'inverse de l'indice de Simpson dans les deux groupes d'échantillons de l'Exemple 3 sous forme de boites à moustaches. La Figure 6a montre la diversité de chaque échantillon et la Figure 6b montre la diversité des deux groupes d'échantillons.

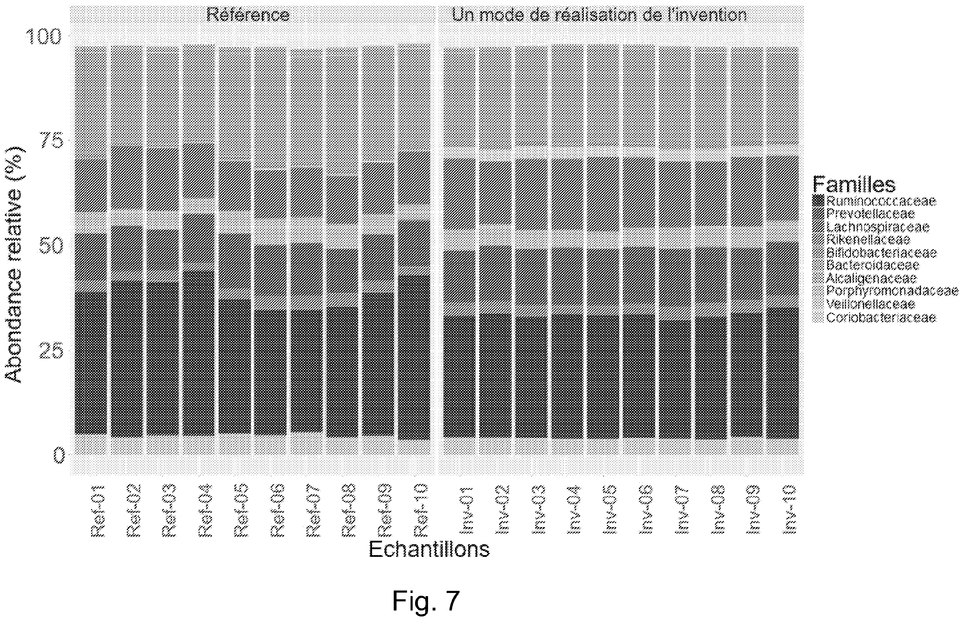

La Figure 7 est un histogramme empilé représentant l'abondance relative des 10 familles bactériennes les plus présentes dans deux groupes d'échantillons de l'Exemple 3. Le groupe à droite (échantillons Inv-01 à Inv-10) est produit selon un mode de réalisation de l'invention. Le groupe à gauche, « Référence » (échantillons Ref-01-Ref-10), est produit selon une méthode de l'art antérieur, Paramsothy et al.

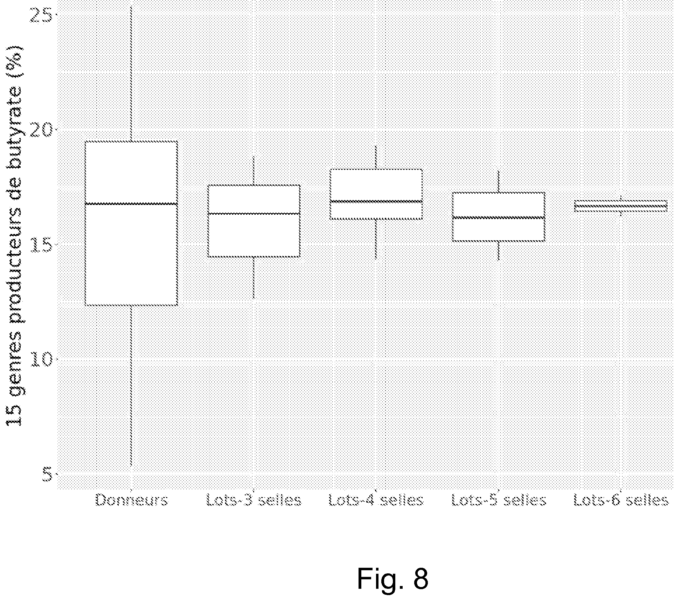

La Figure 8 montre l'abondance relative des 15 genres bactériens producteurs de butyrate, à savoir *Blautia, Faecalibacterium, Alistipes, Eubacterium, Bifidobacterium, Ruminococcus, Clostridium, Coprococcus, Odoribacter, Roseburia, Holdemanella, Anaerostipes, Oscillibacter, Subdoligranulum* et *Butyrivibrio,* dans les selles des donneurs (première boite à gauche), puis dans les lots des mélanges homogènes obtenus à partir des selles de 3, 4, 5 et 6 donneurs respectivement.

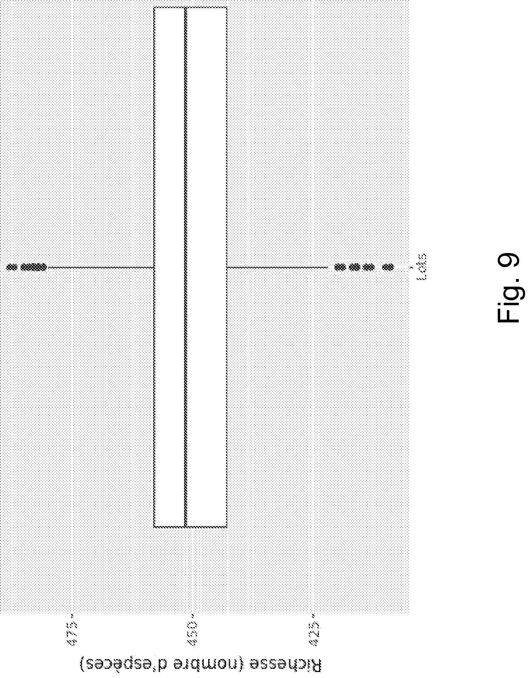

La Figure 9 montre la richesse en espèces dans les différents lots de mélange homogène obtenus à partir des selles de 4, 5 et 6 donneurs.

### Description Détaillée

La présente invention se rapporte à un procédé de collecte et de préparation d'un mélange homogène de microbiote fécal de plusieurs donneurs. L'invention se rapporte également audit mélange homogène susceptible d'être obtenu selon le procédé de l'invention pour son utilisation dans le traitement des dysbioses intestinales et/ou des maladies associées aux dysbioses intestinales par transplantation de microbiote fécal allogénique.

En général, selon l'invention, les donneurs sont des sujets humains sains. Par sujet « sain », on entend un sujet non atteint d'un déséquilibre du microbiote intestinal ou d'une pathologie diagnostiquée/reconnue par le corps médical.

Ainsi, pour sélectionner des donneurs potentiels, un certain nombre de critères ont été définis. Ces critères sont les suivants :
i. ayant entre 18 et 60 ans,
ii. ayant un Indice de Masse Corporel (IMC) entre 18 et 30,
iii. absence d'antécédents personnels de maladies infectieuses graves telles que le SIDA ou l'hépatite virale, de troubles métaboliques et neurologiques, ou de dépression,
iv. absence de prise récente (au cours des 3 mois environ précédant le don) de médicaments pouvant détériorer la composition du microbiote intestinal, tels que des antibiotiques,
v. absence d'apparition récente (au cours des 3 mois environ précédant le don) de symptômes associés à une maladie gastro intestinale, tels que fièvre, diarrhée, nausée, vomissement, douleur abdominale, jaunisse, absence d'historique des maladies infectieuses graves, notamment le SIDA, l'hépatite etc .
vi. absence de voyages récents (au cours des 3 mois environ précédant le don) dans des pays tropicaux,
vii. absence de comportement sexuel à risque,
viii. absence de contact récent (au cours des 3 mois environ précédant le don) avec du sang humain, par exemple, via une blessure, piercing et/ou tatouage,
ix. absence de fatigue chronique récente (au cours des 3 mois environ précédant le don),
x. absence de réaction allergique récente (au cours des 3 mois environ précédant le don),
xi. optionnellement, ayant un régime alimentaire divers.

Les critères de sélection des donneurs sont basés sur ceux utilisés couramment en Europe pour le don de sang, mais avec des critères additionnels spécifiques au don de selles et au contexte de la transplantation de microbiote fécal. Ainsi, des critères (i) à (xi) ont été définis pour sélectionner les donneurs.

Le critère optionnel relatif au régime alimentaire divers a pour objectif d'améliorer la possibilité d'avoir une diversité bactérienne importante dans l'échantillon de microbiote fécal. Il est donc préférable que le donneur ait un régime alimentaire divers.

Par « régime alimentaire divers », on entend un régime alimentaire composé de légumes variés et de différentes céréales (qui permettront l'apport régulier de fibres), mais aussi de fruits et de viandes.

Par « diversité bactérienne », on entend la diversité ou variabilité mesurée au niveau du genre ou de l'espèce. La diversité bactérienne peut être exprimée avec des termes tels que « richesse » (nombre d'espèces observées dans un échantillon), « Indice de Shannon » et « Indice de Simpson ». L'indice de Shannon donne une idée de la diversité spécifique, c'est à dire du nombre d'espèces de l'échantillon (richesse spécifique) et de la répartition des individus au sein de ces espèces (équitabilité spécifique). L'indice de Simpson est dérivé de la richesse et tient compte de l'abondance relative de chaque espèce. Il est compris entre 0 (faible diversité) et 1 (diversité élevée). L'inverse de l'indice de Simpson permet de refléter la diversité (en considérant la richesse et l'abondance relative des espèces comme pour l'indice de Simpson) avec une gamme de valeurs allant de 0 (faible diversité) à l'infini (diversité élevée).

Le procédé selon l'invention, typiquement, comprend une étape a) de prélèvement d'au moins un échantillon de selles, comprenant le microbiote fécal, du sujet donneur. L'étape a) de prélèvement d'au moins un échantillon de selles peut ainsi être réalisée par le donneur lui-même, par exemple, chez lui, ou par un professionnel de santé.

Le prélèvement d'au moins un échantillon de selles est, de préférence, réalisé avec un dispositif de collecte conçu pour cette fonction de sorte que l'échantillon de selles est enfermé dans un environnement anaérobie. On peut ainsi citer le dispositif de collecte décrit dans la demande de brevet WO2016/170290. Ainsi, typiquement, un dispositif de collecte de fèces à domicile est remis aux donneurs sélectionnés avec un guide d'utilisation. De préférence, un échantillon de selles présente une masse d'au moins 20g.

Suite à cette étape de prélèvement, et dans un délai rapide, par exemple, inférieur à 5 minutes suivant le prélèvement, de préférence inférieur à 3 minutes, plus préférentiellement, inférieur à 1 minute, l'échantillon est placé dans un dispositif de collecte étanche à l'oxygène : c'est l'étape b).

Selon un mode de réalisation de l'invention, l'étape de prélèvement d'au moins un échantillon de microbiote fécal est réalisée par le dépôt directement d'un échantillon de selles dans un dispositif de collecte, tel que celui décrit dans la demande de brevet WO2016/170290.

Par la suite, généralement le procédé s'effectue désormais en anaérobiose (en atmosphère anaérobie) ou en confinement où l'exposition à l'air est limitée. L'étape de contrôle c) peut être réalisée en anaérobiose ou en aérobiose ou en confinement où l'exposition à l'air est limitée. Selon un mode de réalisation de l'invention, le prélèvement de l'échantillon pour faire les tests de contrôles se fait en aérobiose. Selon un mode de réalisation de l'invention, les étapes b) et d) à g) du procédé sont réalisées en anaérobiose ou en confinement où l'exposition à l'air est limitée. En limitant l'exposition à l'air, la viabilité des bactéries constitutives du microbiote fécal et présentes dans l'échantillon est ainsi préservée.

De préférence, le dispositif de collecte étanche à l'air se présente sous une forme du type comprenant :
- un conteneur comprenant un corps qui comporte un espace intérieur adapté pour recevoir l'échantillon de microbiote fécal du sujet donneur, et un col qui délimite une ouverture d'accès à l'espace intérieur du corps, et
- un couvercle adapté pour être monté de manière amovible et étanche sur le col du conteneur de manière à obturer l'ouverture d'accès du col et à fermer l'espace intérieur du corps, dans lequel le corps du conteneur est constitué d'une poche souple, et dans lequel au moins l'un parmi le conteneur et le couvercle est pourvu d'un organe d'évacuation adapté pour évacuer au moins une partie des gaz contenus dans l'espace intérieur du corps du conteneur.

De préférence, l'organe d'évacuation du dispositif comprend un passage ménagé au travers de l'un parmi le conteneur et le couvercle, et un élément d'obturation du passage pour empêcher des fluides extérieurs de pénétrer dans l'espace intérieur du corps du conteneur. De préférence, l'organe d'évacuation du dispositif comprend en outre une membrane de filtration microporeuse disposée dans le passage.

Alternativement, le dispositif de collecte étanche à l'air se présente sous une forme du type comprenant :
- un conteneur comprenant un corps qui comporte un espace intérieur adapté pour recevoir l'échantillon de microbiote fécal du sujet donneur, et un col qui délimite une ouverture d'accès à l'espace intérieur du corps, et
- un couvercle adapté pour être monté de manière amovible et étanche sur le col du conteneur de manière à obturer l'ouverture d'accès du col et à fermer l'espace intérieur du corps,
- dans lequel l'espace intérieur du corps du conteneur comprend éventuellement un dispositif chimique neutralisant l'oxygène.

Par exemple, pour réaliser les étapes a) à d) (voire e)) du procédé, selon un mode de réalisation de l'invention, on peut utiliser un dispositif de collecte muni d'au moins un dispositif annexe par exemple, un dispositif annexe d'alimentation adapté pour alimenter l'espace intérieur en fluide (par exemple, la solution ajoutée à l'étape d)), tel que décrit dans le document WO 2016/170290.

Par ailleurs, le dispositif annexe peut également être un tube d'analyse, utilisé pour sortir un échantillon pour analyse (par exemple, un contrôle qualitatif selon l'étape c)).

De préférence, le dispositif de collecte étanche à l'air est utilisé pour les étapes a) et b) : le prélèvement de l'échantillon de l'étape a) s'effectue directement dans ledit dispositif, notamment dans le conteneur, et la fermeture du dispositif, notamment grâce au couvercle, place l'échantillon en atmosphère sans oxygène (étape b)).

En particulier, le dispositif mentionné ci-dessus, utilisé à l'étape b), permet de réaliser les étapes b), d) et e) en anaérobiose.

De façon optionnelle, une étape de transport peut ainsi avoir lieu. Cette étape de transport permet de rapatrier l'échantillon du lieu de prélèvement au laboratoire, pour traitement ultérieur et analyse.

Après l'étape b), de préférence, en moins de 24 heures après l'étape b), l'étape c) de contrôle qualitatif est réalisée sur les échantillons prélevés. Ce contrôle qualitatif a pour objet d'éliminer des échantillons de microbiote fécal qui ne répondent pas aux critères qualitatifs prédéfinis. Ainsi les prélèvements retenus après le contrôle sont considérés comme acceptables pour former le mélange homogène de microbiote fécal souhaité.

De manière générale, les critères de qualité qui sont contrôlés comprennent :
- consistance de l'échantillon entre 1 et 6 selon l'échelle de Bristol, par inspection visuelle,
- absence de sang et d'urine dans l'échantillon,

Les critères peuvent également inclure :
- absence des bactéries suivantes : *Campylobacter, Clostridium difficile (toxin A*/*B), Salmonella, Yersinia enterocolitica, Vibrio sp., Shigo-like toxin-producing, E.coli (STEC) stxl*/*stx2,* des bactéries multi résistantes : bactéries productrices de Bêta-lactamase à spectre étendu (BLSE) - Entérocoques résistant à la vancomycine et aux glycopeptides (ERV, GRE), *Listeria monocytogenes,* et les bactéries résistantes aux carbapénèmes,
- absence des parasites suivants : *Cryptosporidium parvum, Cyclosporasp, Entamoeba histolytica, Giardia lamblia, Blastocystis hominis, Helminths, Strongyloides stercoralis, Isospora sp., Microsporidies et Dientamoeba fragilis,*
- absence des virus suivants : Adenovirus F40/41, Astrovirus, Norovirus, Rotavirus A, Sapovirus et Picornavirus (Aichi Virus et enterovirus),
- absence des bactéries suivantes : *E.coli enteroaggregative* (EAEC), E*.coli enteropathogenic* (EPEC), E*.coli enterotoxigenic* (ETEC) It/st, *Shigella*/*Enteroinvasive E.coli* (EIEC) et *Plesiomonas shigelloides.*

Le contrôle de consistance de l'échantillon est réalisé, de manière générale, par inspection visuelle. Si l'échantillon a un aspect entre 1 et 6, de préférence 5, selon l'échelle de Bristol [Lewis, S.J. ; Heaton, K.W. (September 1997). "Stool form scale as a useful guide to intestinal transit time". Scand. J. Gastroenterol.], il est acceptable et sera retenu.

Le contrôle d'absence de sang et d'urine dans l'échantillon peut être réalisé, par inspection visuelle ou par des autres moyens. Par exemple, des tests immunologiques rapides peuvent être utilisés. Par exemple, le test OC Sensor^{®} (disponible chez MAST Diagnostic en France), un test immunologique quantitatif qui détecte l'hémoglobine grâce à des anticorps spécifiques de la globine humaine.

Si la présence du sang et/ou d'urine est constatée, l'échantillon est éliminé.

Selon un mode de réalisation de l'invention, un contrôle de l'absence de certains parasites, virus et bactéries de l'échantillon peut être également réalisé. Typiquement, un contrôle d'absence de certains parasites, virus et bactéries de l'échantillon est réalisé une fois par semaine, par donneur. Cela signifie que, typiquement, pour un donneur qui fournit, par exemple, cinq échantillons dans la semaine, un échantillon sur cinq sera contrôlé. Selon un mode de réalisation de l'invention, l'étape de contrôle d'absence de certains parasites, virus et bactéries de l'échantillon est réalisée sur chaque échantillon.

Les contrôles d'absence de certaines bactéries, de certains parasites et de certains virus des échantillons sont réalisés selon les méthodes connues de l'homme de métier.

De préférence, les bactéries suivantes doivent être absentes de l'échantillon : *Campylobacter, Clostridium difficile (toxin A*/*B), Salmonella, Yersinia enterocolitica, Vibrio sp., Shigo-like toxin-producing E.coli (STEC) stxl*/*stx2, Listeria monocytogenes* et les bactéries multi résistantes, telles que les bactéries gram négatif produisant de la Bêta-lactamase à spectre étendu (BLSE) et les Entérocoques résistant à la vancomycine et aux glycopeptides (ERV, GRE), *Listeria monocytogenes,* bactéries résistantes aux carbapénèmes, *E.coli enteroaggregative* (EAEC), E*.coli enteropathogenic* (EPEC), E*.coli enterotoxigenic* (ETEC) It/st, *Shigella*/*Enteroinvasive E.coli* (EIEC) et *Plesiomonas shigelloides.*

De préférence, les bactéries citées ci-dessus sont des pathogènes dont la présence dans l'échantillon de microbiote fécal recueilli exclura le donneur dudit échantillon de la sélection.

De manière similaire, de préférence, les parasites suivants doivent être absents de l'échantillon de microbiote fécal : *Cryptosporidium, Cyclospora cayetanensis, Entamoeba histolytica* et *Giardia lamblia, Blastocystis hominis, Helminths, Strongyloides stercoralis, Isospora sp., Microsporidies* et *Dientamoeba fragilis.*

De manière similaire, de préférence, les virus suivants doivent être absents de l'échantillon de microbiote fécal : Adenovirus F40/41, Astrovirus, Norovirus, Rotavirus A, Sapovirus et Picornavirus (Aichi Virus et enterovirus).

Des contrôles de présence de bactéries, de parasites et de virus sont réalisés selon des méthodes connues de l'homme de métier. On peut citer comme exemples les méthodes suivantes : culture dans des conditions sélectives, détection des bactéries, des parasites ou des virus avec des anticorps, amplification (avec par exemple, PCR) et analyse des séquences d'ADN présentes dans des échantillons. Comme système d'analyse des séquences d'ADN, on peut citer le FilmArray^{®} de BioMerieux (France) un système automatisé qui peut être utilisé pour la détection de bactéries, de parasites et de virus. Par exemple, on peut détecter les parasites suivants au moyen de ce système : *Cryptosporidium, Cyclospora cayetanensis, Entamoeba histolytica* et *Giardia lamblia.* On peut également citer la série de tests PCR « AlIplex-GI » disponible de Eurobio (France).

D'autres parasites tels que *Blastocystis hominis,* Isopora sp, *Microsporidies* et *Dientamoeba fragilis* peuvent être détectés par examen microscopique avec concentration de l'échantillon si nécessaire. *Strongyloides stercoralis* peut être détecté avec, par exemple coloration élective, après concentration de l'échantillon si nécessaire.

Des bactéries multi résistantes, telles que BLSE, ERV et GRE, peuvent être détectées par cultures dans des conditions spécifiques, par exemple le milieu ESBL, VRE ou ALOA disponibles dans le commerce, par exemple de BioMerieux.

Pour certaines espèces de bactéries et de virus, leur absence de l'échantillon n'est pas requise. Selon un mode de réalisation de l'invention, la présence des bactéries suivantes n'est pas un critère pour l'exclusion de l'échantillon : *E.coli enteroaggregative* (EAEC), E*.coli enteropathogenic* (EPEC), E*.coli enterotoxigenic* (ETEC) It/st, *Shigella*/*Enteroinvasive E.coli* (EIEC) et *Plesiomonas shigelloides.* Selon un mode de réalisation de l'invention, la présence du virus EBV n'est pas un critère d'exclusion car ce virus est prévalant aujourd'hui dans la population humaine générale.

De manière générale, les échantillons retenus après l'étape de contrôle c) qui comprend un contrôle d'absence de sang et d'urine dans l'échantillon, et, éventuellement, un contrôle de l'absence de certains parasites, virus et bactéries dans l'échantillon, passent à l'étape d). L'étape d) comprend l'ajout d'un diluant cryoprotecteur.

De manière générale, pour l'étape d), les échantillons sont transformés séparément en des inocula liquides en ajoutant du diluant cryoprotecteur. De manière générale, une solution aqueuse saline comprenant au moins un agent cryoprotecteur et/ou un agent de charge est ajoutée à chacun des échantillons retenus après l'étape de contrôle c).

Tout diluant/cryoprotecteur approprié peut être utilisé pour la préparation des inocula. De préférence, des polyols ou des di-, tri- ou polysaccharides, ou un mélange de ceux-ci peuvent être utilisés. Comme polyol, on peut citer le glycérol, le mannitol, le sorbitol, le propylène glycol ou l'éthylène glycol. Comme di-, tri- ou polysaccharides, on peut citer des dimères, trimères, tétramères et pentamères d'unités différentes ou identiques, lesdites unités étant choisies parmi le glucose, le fructose, le galactose, le fucose et l'acide N acétylneuraminique. Parmi les disaccharides pouvant être utilisés, on peut citer le tréhalose ou l'un de ses analogues ou saccharose. De préférence, le cryoprotecteur est choisi parmi le glycérol, le mannitol, le sorbitol, le propylène glycol, l'éthylène glycol, le tréhalose et ses analogues, le saccharose, le galactose-lactose et leurs mélanges. Plus préférablement, le cryoprotecteur est le galactose-lactose ou le tréhalose.

Typiquement, la quantité de cryoprotecteur présente dans la solution aqueuse saline est comprise entre 3 et 30% en poids par rapport au volume total de l'inoculum final (p / v), de préférence entre 4 et 20% (p / v).

Comme agents de charge, on peut citer par exemple les hydrolysats partiels d'amidon, notamment de blé ou de maïs, ainsi que les hydrolysats partiels de féculents, par exemple, de pomme de terre, contenant de grandes quantités de maltodextrine. De préférence, l'agent de charge est un mélange de maltodextrines, dans lequel la maltodextrine est présente entre 3 et 30%, de préférence entre 4 et 20% (par rapport au volume total de l'inoculum final poids / volume).

Selon un mode de réalisation de l'invention, le diluant/cryoprotecteur est une solution saline aqueuse comprenant au moins un cryoprotecteur et un agent de charge.

Typiquement, la solution contient de l'eau et des sels physiologiquement acceptables. Typiquement, la solution contiendra des sels de calcium, de sodium, de potassium ou de magnésium avec des fers de chlorure, de gluconate, d'acétate ou d'hydrogénocarbonate. La solution aqueuse saline peut éventuellement contenir également au moins un antioxydant. L'antioxydant peut être choisi parmi l'acide ascorbique et ses sels, les tocophérols, la cystéine et ses sels, notamment le chlorhydrate, et leurs mélanges. Préférentiellement, la solution aqueuse saline comprend au moins un sel choisi parmi le chlorure de sodium, le chlorure de calcium, le chlorure de magnésium, le fluorure de potassium, le gluconate de sodium et l'acétate de sodium et éventuellement au moins un antioxydant choisi parmi le L-ascorbate de sodium, le tocophérol, le chlorhydrate de cystéine monohydraté et leurs mélanges. Typiquement, le sel est présent dans la solution aqueuse saline à une concentration comprise entre environ 5 et 20 g / L, de préférence entre 7 et 10 g / L (par rapport au volume total de l'inoculum final). Typiquement, l'antioxydant est présent dans la solution aqueuse saline en une quantité comprise entre 0,3 et 1% en poids / volume, de préférence entre 0,4 et 0,6% en poids / volume (par rapport au volume total de l'inoculum final).

De manière générale, la solution aqueuse saline comprenant au moins un agent cryoprotecteur et/ou un agent de charge est ajoutée à l'échantillon de microbiote fécal avec le rapport de poids (g) / volume (ml) compris entre 1 : 0,5 et 1 : 10, de préférence entre 1 : 2 et 1 : 8, plus préférentiellement 1 : 4. Un rapport poids/volume échantillon : solution égal à 0,5 poids : 10 volumes signifie que l'échantillon est mélangé à 0,5 poids (par exemple 0,5g) pour 10 volumes de solution (par exemple 10ml).

De préférence, l'étape d) d'ajout d'une solution aqueuse saline comprenant au moins un agent cryoprotecteur et/ou un agent de charge est réalisée en anaérobiose, ou en confinement où l'exposition à l'air est limitée. Selon un mode de réalisation de l'invention, la solution saline comprenant au moins un agent cryoprotecteur et/ou un agent de charge est ajoutée dans le dispositif annexe du dispositif de collecte mentionné ci-dessous, et la solution est ajoutée à l'échantillon de selles via une conduite fermée. Le mélange de l'échantillon avec au moins une solution aqueuse saline comprenant au moins un agent cryoprotecteur et/ou un agent de charge peut notamment être réalisé par malaxage, afin d'obtenir un mélange homogène.

Les échantillons obtenus après cette étape de dilution sont ensuite filtrés, à l'étape e). De préférence, l'étape de filtration se réalise avec un (ou plusieurs, ayant des pores de plus en plus réduits en taille) filtre(s) comprenant des pores de diamètre inférieur ou égal à 0,5mm, de préférence inférieur ou égal à 265 µm. Dans le cas où plusieurs filtres sont utilisés, la taille des pores diminue progressivement. De préférence, les premiers filtres utilisés comprennent des pores de diamètre inférieur ou égal à 2mm, de préférence inférieur ou égal à 1mm. De préférence, le dernier filtre utilisé comprend des pores de diamètre inférieur ou égal à 0,5mm, de préférence inférieur ou égal à 265 µm. Ainsi, on obtient les inocula individuels. De préférence, cette étape de filtration e) est réalisée en anaérobiose, ou en confinement où l'exposition à l'air est limitée. Lors de la filtration, l'échantillon issu de l'étape d) peut être poussé au travers des filtres manuellement, par action mécanique, par gravité, par vacuum ou par d'autres moyens appropriés.

Selon un mode de réalisation de l'invention, la même quantité (en termes de poids) de selles par donneur est utilisée pour former l'inoculum, c'est-à-dire pour réaliser les étapes d) et e). On peut citer comme quantité appropriée, par exemple, 25-80g, de préférence, 40g. Ainsi, chaque inoculum individuel est produit en utilisant la même quantité d'échantillon de microbiote fécal.

L'étape f) consiste à regrouper les inocula issus de l'étape de filtration e). En particulier, typiquement, les inocula individuels sont transférés dans un conteneur, de préférence, une poche souple. Typiquement, le volume de ce conteneur qui regroupe des inocula est de 1L à 5L, de préférence de 3L ou de 5L. Ce volume peut être plus important selon l'échelle d'industrialisation du procédé. Le transfert peut être réalisé manuellement ou en utilisant des moyens mécaniques De préférence, le transfert des inocula est effectué en utilisant des moyens mécaniques, par exemple, une seringue, plus préférentiellement, avec une pompe péristaltique. Des pompes péristaltiques appropriées sont disponibles dans le commerce, par exemple, chez Interscience (France).

Une fois regroupés, les inocula sont mélangés de manière à former un mélange homogène (étape d'homogénéisation g)). Ce mélange homogène est ainsi considéré comme « un lot » de microbiote fécal. Le mélange des inocula peut être réalisé par tous moyens. Le mélange des inocula peut être réalisé manuellement ou par des moyens mécaniques connus de l'homme de métier. Par exemple, on peut citer un plateau d'agitation, disponible, par exemple, chez Stuart (Angleterre).

En général, un taux d'agitation de 80-200 rpm, de préférence entre 90 et 150 rpm, plus préférentiellement 100-135 rpm, peut être utilisé. En général, l'homogénéisation peut avoir lieu pendant une période de temps comprise entre 10 minutes et 2 heures, de préférence, 20 minutes et 1 heure, plus préférentiellement pendant 30 minutes. La durée dépend de la vitesse d'agitation. L'homme du métier sait déterminer le temps d'homogénéisation nécessaire en fonction de la méthode d'homogénéisation qu'il utilise. Un test colorimétrique peut être utilisé pour vérifier si le mélange est homogène. Une inspection visuelle peut aussi être utilisée. De préférence, un test colorimétrique suivi par une inspection visuelle sont réalisés pour déterminer si le mélange est homogène.

L'étape d'homogénéisation peut se faire à une température entre 2 et 25°C, de préférence entre 2°C et 8°C, plus préférentiellement à environ 4°C.

Selon un mode de réalisation de l'invention, une étape d'analyse peut être réalisée sur le mélange homogène obtenu après l'étape g), avant que le mélange soit stocké ou lyophilisé (voir détails ci-dessous). Le pH et le pO2 peuvent être mesurés. Les méthodes connues de l'homme de métier sont utilisées pour effectuer ces mesures. Typiquement, le pO2 du mélange est à moins de 10%, de préférence à moins de 5% ; typiquement le pH est entre 4 et 7, de préférence entre 4,5 et 6,5.

De préférence, il se passe moins de 76 heures entre le début de l'étape a) et la fin de l'étape g).

De manière générale, le produit final (le mélange homogène) répond aux spécifications suivantes :
L'aspect : suspension colorée, homogène, qui a une couleur jaune/marron.

La viabilité : supérieure à 20%, de préférence, supérieure à 40% pour lyophilisation.

Le nombre d'événements (de bactéries) : supérieur à 10⁹ bactéries/ml.

La diversité bactérienne : l'inverse de l'indice de Simpson supérieur ou égal à 4. De préférence, l'inverse de l'indice de Simpson du mélange des inocula est supérieur ou égal à 10, plus préférentiellement, supérieur à 15, encore plus préférentiellement, supérieur à 20.

De manière générale, le mélange homogène est transféré (étape h) de transfert) pour stockage ou lyophilisation. Ainsi le mélange peut être transféré dans des poches :
- pour stockage à une température d'environ -50°C à -80°C, de préférence à environ -80°C, pour utilisation du mélange au-delà de 16 heures, ou
- pour stockage à une température entre 2 à 6 °C pour utilisation sous 16 heures environ, ou
- pour stockage à une température entre 10 à 25 °C pour utilisation du mélange dans les quatre prochaines heures. Au-delà de quatre heures à température ambiante, le nombre de bactéries augmente et l'homogénéité de l'inoculum peut être réduite.

Sinon, le mélange homogène peut être transféré dans un dispositif de lyophilisation pour lyophilisation ultérieure ou immédiate.

Selon un mode de réalisation de l'invention, une étape d'analyse i) peut être réalisée sur le mélange homogène obtenu après l'étape de transfert l'étape h) du mélange homogène dans son lieu de stockage (typiquement une poche) ou dans son lieu de lyophilisation (typiquement dans un dispositif de lyophilisation). Spécifiquement, cette étape d'analyse comprend une inspection visuelle, des mesures de viabilité, une analyse taxonomique et des mesures de nombres d'évènements /ml. Cette étape d'analyse a pour but de déterminer si l'échantillon répond aux critères qualitatifs pour son utilisation en thérapie TMF.

Typiquement, le mélange a une couleur jaune/brun. Typiquement, la viabilité doit être >20%. Selon un mode de réalisation de l'invention, la viabilité est de préférence > 40%. Si le mélange doit être lyophilisé, il est préférable que la viabilité soit > 40%. En général, la concentration cellulaire mesurée par cytométrie en flux est supérieure à 10⁶, de préférence, supérieure à 10⁷ bactéries /ml, et plus préférentiellement supérieure à 10⁹ bactéries /ml. En termes d'analyse taxonomique, en général, il est préférable que l'indice de Shannon soit supérieur à 3,5, de préférence supérieur à 4.

La bonne qualité du mélange issu du procédé selon un mode de réalisation de l'invention a été démontrée par les demandeurs. Les résultats d'une évaluation qualitative du procédé, selon un mode de réalisation de l'invention, réalisée avec six selles fraîches de donneurs sains, sont montrés dans l'Exemple 1. Des tests de viabilité du microbiote ont été effectués sur chaque inoculum avant l'étape f) de regroupement et après l'étape g) d'homogénéisation.

Les demandeurs ont constaté que le mélange homogène constitué des inocula regroupés avait une viabilité bactérienne qui lui est propre, et qui est plus élevée que ce que l'on peut attendre.

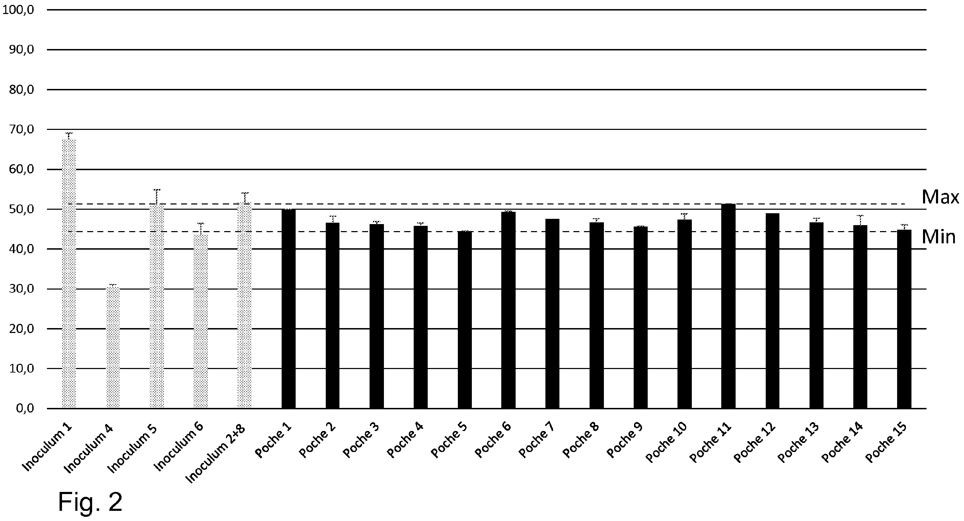

La Figure 2 est un histogramme représentant le pourcentage de viabilité du microbiote des inocula de l'Exemple 1 (inoculum 1, 4, 5, 6 et 2 + 8) et du mélange d'inoculum (mélange homogène) dans des poches 1 à 15 d'inoculum destinées à être stockées. La Figure 2 démontre que les inocula individuels (inoculum 1, 4, 5, 6, 2 + 8) n'ont pas la même viabilité individuelle, mais que le mélange d'inoculum a sa propre viabilité, différente des inocula individuels. De plus, le procédé selon l'invention assure une bonne reproductibilité des poches de mélange final, puisque la viabilité du microbiote est approximativement la même pour chaque poche (supérieure à 40%).

Une analyse statistique sur les poches de mélange homogène indique qu'il n'existe pas de différence significative selon un test-t et selon un test de rang, que ce soit pour la viabilité du microbiote que pour le nombre d'événements/µL. Ainsi, les demandeurs ont montré que toutes les poches de mélange préparées sont homogènes entre elles.

Les résultats d'une évaluation qualitative du procédé, selon un mode de réalisation de l'invention, réalisée avec quatre lots (Lot N°1 à Lot N°4) différents de selles fraîches de donneurs sains, sont montrés dans l'Exemple 2. Le lot N° 4 est le même que celui utilisé pour l'Exemple 1 (et illustré dans la Figure 2). Dans l'Exemple 2, le nombre de donneurs est de 2, 7, 4 et 6 pour le lot N°1, le lot N°2, le lot N°3 et le lot N° 4, respectivement. Les demandeurs ont mesuré la viabilité et la diversité bactérienne des inocula individuels (à l'issue de l'étape e)), des inocula regroupés homogénéisés (à l'issue de l'étape g)) et des poches remplies avant stockage.

La Figure 3 démontre encore que les inocula individuels varient, mais que le mélange d'inoculum a sa propre viabilité, différente de l'inoculum individuel. La viabilité des lots N°1 à 4 se situe entre 46,1% et 60,1%, ce qui est excellent.

La Figure 4a démontre la richesse des échantillons. Le coefficient de variation (CV) a été calculé pour chaque inoculum. Le CV (coefficient de variation ou écart relatif) est une mesure de dispersion des données autour de la moyenne. Il s'agit du calcul de rapport de l'écart-type sur la moyenne. Le CV permet de calculer le degré de variation d'un échantillon à un autre. Plus la valeur du CV est petite, plus les valeurs sont homogènes ou stables. Le CV des inocula individuels des quatre lots varie entre 16% et 81%. Cette différence est normale parce qu'il est connu que des microbiotes individuels sont très différents. Pour chaque inoculum regroupé (à l'issue de l'étape g)), le coefficient de variation, par lot, est entre 0,3% et 2%. Cette faible variation indique que le mélange des inocula pour chaque lot est homogène et stable.

La richesse mesurée au niveau de l'espèce ou du genre est nettement supérieure dans les inocula regroupés, en comparaison avec les inocula individuels. En moyenne, la richesse augmente de 64% dans le lot N° 4 et de 147% dans les lots N° 1 et 3 en comparaison avec les inocula individuels.

En moyenne, la richesse mesurée au niveau du genre de l'inoculum regroupé augmente de 25% pour le lot N° 4 et de 61 % pour les lots N° 1 et 3 en comparaison avec les inocula individuels. La richesse des lots 1 à 4 est surprenante, et ne pourra pas être déduite des richesses individuelles des inocula individuels ; c'est-à-dire que cette richesse des mélanges d'inocula (les inocula regroupés) n'est pas la moyenne, ni directe ni pondérée par la masse des inocula individuels, ni même par fraction des richesses individuelles mesurées. Ce résultat est inattendu.

Le pourcentage de Proteobacteria présente dans les quatre inocula regroupés a été mesuré (voir tableau 5). Les valeurs sont de 5,5%, 3,7%, 3,1% et 3,4% pour les lots N° 1, 2, 3 et 4 respectivement.

En conclusion, le regroupement des inocula résulte en une augmentation inattendue de la richesse du microbiote. Ainsi, le mélange d'inocula est un échantillon de microbiote fécal ayant des caractéristiques qui lui sont propres.

La Figure 4b démontre que le procédé implique une standardisation des produits (mélange homogène) obtenus pour un lot, et destinés à être utilisés en clinique. La Figure démontre que la similarité Bray Curtis entre les poches (contenant l'inoculum regroupé) pour chaque lot est supérieure à 96%. Ce résultat montre qu'en termes de profil taxonomique, les poches d'inoculum (le mélange homogène) sont homogènes.

La diversité bactérienne a été mesurée en utilisant l'inverse de l'indice de Simpson et l'indice de Shannon (Tableau 5).

Les résultats indiquent que l'inverse de l'indice de Simpson pour les donneurs varie entre 5 et 30. Cette différence est normale puisque chaque microbiote est différent. D'après les données présentées dans le Tableau 5, nous pouvons observer que l'inverse de l'indice de Simpson pour l'inoculum regroupé se situe entre 20,2 et 27,2. La diversité bactérienne de l'inoculum regroupé (après l'étape g)) est plus élevée que celle des donneurs individuels, (à l'exception du lot N°3 où la diversité d'un donneur est supérieure à celle de l'inoculum regroupé).

L'indice de Shannon est entre 2,4 et 4,2 pour les donneurs. Cette différence est normale puisque chaque microbiote est différent. L'indice de Shannon pour l'inoculum regroupé est compris entre 4 et 4,50 (voir Tableau 5).

Nous pouvons également observer que pour chaque lot la diversité de l'inoculum regroupé est plus élevée que celle des donneurs individuels. Les valeurs obtenues, comme pour les richesses, ne pouvaient être prédites et constituent pour chacune une caractéristique propre du mélange homogène.

Les inventeurs ont réalisé des tests comparatifs pour démontrer que, de manière générale, le mélange d'inocula susceptible d'être obtenu selon la méthode de l'invention présente des caractéristiques avantageuses par rapport aux échantillons de microbiote fécal obtenus selon des méthodes connues, et en particulier, les méthodes avec des multi-donneurs. Ainsi, il est décrit dans l'Exemple 3 comment les inventeurs ont caractérisé des échantillons produits selon la méthode décrite dans l'art antérieur [Paramsothy S., et al. (2017), Multidonor intensive faecal microbiota transplantation for active ulcerative colitis: a randomised placebo-controlled trial, Lancet, Mar 25; 389(10075): 1218-1228] et comment ils les ont comparés avec ceux produits selon un mode de réalisation de l'invention. Les deux méthodes de production sont décrites dans la Figure 5. Les échantillons produits selon un mode de réalisation de l'invention présentaient une meilleure viabilité et une meilleure diversité par rapport à ceux produits selon la méthode de l'art antérieur.

La viabilité des produits a été mesurée. La viabilité de l'inoculum regroupé selon un mode de réalisation de l'invention évolue de 41,8 % en moyenne sur 5 échantillons à 41,4% après un mois de stockage à -80°C +/-10°C. Les échantillons obtenus selon la méthode de Paramsothy et al. évoluent de 38,5% en moyenne à 37,5%. Cette évolution de viabilité n'est pas significative selon le test de Wilcoxon avec les valeurs de p de 0.462 pour les échantillons selon l'invention et une valeur de p de 0.1732 pour les échantillons de référence (selon Paramsothy et al.).

Cependant, le test de Wilcoxon montre une différence significative entre la viabilité entre les deux groupes d'échantillons à T0 (jour 0), avec une valeur de p égale à 0,01193, ainsi qu'à 1 mois (p=0.007937).

De plus, le coefficient de variation (CV) pour les échantillons préparés selon la méthode de l'invention à 0 jours est de 0,010 comparé avec 0,034 pour des échantillons de référence selon l'art antérieur (Paramsothy et al.). A un mois, le CV des échantillons préparés selon la méthode de l'invention est de 0,018 comparé à 0,028 pour les échantillons de référence. Comme pour l'Exemple 1, les inventeurs ont démontré que les échantillons produits pour l'Exemple 2 possèdent une très faible variation qui indique que le mélange des inocula pour chaque lot est homogène et stable. Par comparaison, les échantillons de référence montrent un CV plus important.

Les inventeurs ont analysé la diversité microbienne des échantillons de l'Exemple 3 en analysant l'ADNr-16S des échantillons. La diversité est exprimée avec l'inverse de l'indice de Simpson qui prend en compte à la fois la richesse (le nombre d'OTU/espèces identifiées) et leur abondance relative respective.

La Figure 6 montre des valeurs de l'inverse de l'indice de Simpson comprises entre 30,96 et 33,69 (médiane = 32,56) pour les échantillons préparés selon un mode de réalisation de l'invention et entre 13,70 et 23,18 (médiane = 19,31) pour les échantillons « Référence » préparés selon Paramsothy et al. La diversité observée est donc supérieure dans les échantillons préparés selon un mode de réalisation de l'invention. Les échantillons préparés selon un mode de réalisation de l'invention présentent par ailleurs une meilleure homogénéité que les échantillons « Référence », ce qui est attesté par le coefficient de variation, 2,7% pour les premiers contre 16,1% pour les derniers. Le test de rang évalue une différence « très hautement significative » entre les deux méthodes (p<2,2e-16).

La Figure 7 montre l'abondance relative des 10 familles bactériennes les plus présentes dans deux groupes d'échantillons de l'Exemple 3. Les échantillons Inv-01 à Inv-10 présentaient une homogénéité nettement meilleure par rapport aux échantillons Ref-01-Ref-10 produits selon une méthode de l'art antérieur, Paramsothy et al. Bien que visuelle, cette représentation permet d'observer le niveau d'hétérogénéité généré par la méthode Paramsothy et al., ainsi que les écarts d'abondance pour certaines familles entre les deux procédés.

Les écarts d'homogénéité, de diversité et d'abondance se concrétisent aux différents niveaux de l'échelle taxonomique. D'ailleurs, comme le montre le Tableau 6 de l'Exemple 3, les inventeurs ont constaté que certaines espèces bactériennes, reconnues comme étant favorables pour la santé sont mieux conservées dans les échantillons Inv-01 à Inv-10 en comparaison avec les échantillons Ref-01-Ref-10. Par exemple, pour le phylum des Actinobactéries, l'abondance relative chute très significativement en utilisant la méthode selon Paramsothy et al. puisque cette abondance médiane est de 3,6% pour les échantillons Inv-01-Inv-10 contre 0,9% pour les échantillons Ref-01 à Ref-10. En termes d'homogénéité, les inventeurs ont observé des coefficients de variation respectifs de 7,8% et 14,5%. D'ailleurs, dans la Figure 7, on observe une diminution importante de l'abondance relative des Coriobacteriaceae, famille du phylum des Actinobacteria, entre les échantillons de l'invention et les échantillons de référence.

Des résultats similaires sont observés au niveau du genre *Prevotella* (genre relié au phylum des Bacteroidetes) et confirment les différences tant en maintien de certains taxa qu'en matière d'homogénéité des deux méthodes. L'abondance relative de *Prevotella* est plus élevée dans les échantillons Inv-01-Inv-10 (médiane à 13%) par rapport aux échantillons Ref-01 à Ref-10 (médiane à 11%). En termes d'homogénéité, les inventeurs ont observé des coefficients de variation respectifs de 3,3% et 8 ,5%.

Enfin, le genre *Bifidobacterium* (appartenant aux Actinobactéries) confirme également les résultats déjà observés aux autres niveaux taxonomiques. *Bifidobacterium* voit son abondance relative diminuer de façon significative (0,9% vs 0,6%) et l'homogénéité entre les échantillons générés par la méthode Paramsothy et al. est moins importante que pour ceux générés par la méthode selon un mode de réalisation de l'invention (voir le Tableau 6 de l'Exemple 3).

Ces éléments montrent donc que, de manière générale, la méthode revendiquée permet une meilleure conservation de ce phylum associée à une bien meilleure homogénéité au niveau des échantillons fabriqués pour chaque lot, surtout les échantillons produits selon un mode de réalisation de l'invention par rapport à ceux produits selon la méthode de l'art antérieur. Cette meilleure homogénéité est primordiale pour la caractérisation du produit final en tant que produit pharmaceutique. Ainsi, un patient qui reçoit plusieurs échantillons de microbiote fécal du même lot, reçoit chaque fois un produit homogène ayant la même diversité et la même viabilité microbienne. Cette reproductibilité est assurée grâce à la méthode utilisée pour produire les échantillons.

En conclusion, l'analyse métagénomique (séquençage de l'ADNr 16S) de l'Exemple 3 a permis de démontrer que les échantillons obtenus selon un mode de réalisation de l'invention présentaient:
- une meilleure conservation de la diversité illustrée par l'inverse de l'indice de Simpson très supérieur,
- une conservation significativement supérieure de certains taxa d'intérêt comme les Actinobactéries, qui incluent le genre bien connu *Bifidobacterium,*
- un niveau d'homogénéité entre les échantillons plus important par rapport à celui obtenu pour l'ensemble des échantillons produits avec la méthode selon Paramsothy et al.

La méthode selon l'invention, de manière générale, a une meilleure reproductibilité par rapport à celle de l'art antérieur, ce qui est très important pour un procédé destiné à la fabrication de médicaments, pour lesquels la notion de lot homogène et reproductible est à la base de la réglementation, Les valeurs de richesse mesurées pour les différents lots (voir Figure 9) montrent en effet une grande reproductibilité entre les lots avec un coefficient de variation de 3,5%. Ainsi, ces résultats démontrent qu'il existe une homogénéité de microbiote fécal inter-lots, ainsi qu'intra-lot. Les inventeurs ont observé que le mélange d'inocula ainsi obtenu a un profil taxonomique et une viabilité bactérienne supérieurs à ceux de certains inocula individuels qui impliquent qu'il est parfaitement adapté pour une utilisation dans le TMF (Transfert de Microbiote Fécal) allogénique. Le mélange d'inocula obtenu selon le procédé de l'invention est supérieur en qualité pour utilisation en TMF car il est caractérisé par une viabilité haute et stable, ainsi qu'une très haute diversité. Ces caractéristiques permettent un potentiel de recolonisation de microbiote adapté plus élevé par rapport à ce qu'on obtiendrait en utilisant un inoculum individuel.

Selon un mode de réalisation préférée de l'invention, le procédé de préparation des échantillons de microbiote permet d'obtenir un mélange homogène de microbiote comprenant 15 genres bactériens producteurs de butyrate, à savoir *Blautia, Faecalibacterium, Alistipes, Eubacterium, Bifidobacterium, Ruminococcus, Clostridium, Coprococcus, Odoribacter, Roseburia, Holdemanella, Anaerostipes, Oscillibacter, Subdoligranulum* et *Butyrivibrio.* Ce mode de réalisation est illustré dans l'Exemple 4.

L'Exemple 4 décrit les expériences visant à obtenir des mélanges homogènes de selles de huit donneurs répondant aux critères de sélection (l'étape c)). Chaque selle ayant passé le contrôle qualité a ensuite été traitée séparément par ajout de diluant cryoprotecteur, puis filtration. Les inocula individuels obtenus un même jour ont ensuite été mélangés de sorte à obtenir des lots de produits homogènes.

L'analyse des selles des donneurs et des lots de mélanges produits montre que la concentration des 15 genres bactériens précités producteurs de butyrate est stable dans les mélanges, quel que soit le nombre de selles utilisées pour le produire. L'analyse montre également que l'abondance de ces 15 genres bactériens producteurs de butyrate se standardise quand le nombre de selles utilisées augmente (Figure 8 et Tableau 7). De plus, les 15 genres bactériens producteurs de butyrate ne se retrouvent pas tous dans les selles individuelles des donneurs, alors que tous les lots fabriqués avec un mélange d'au moins quatre selles les contiennent tous.

Selon un mode de réalisation préféré de l'invention, le procédé de préparation d'un mélange homogène de microbiote fécal provient d'au moins quatre donneurs. Selon un mode de réalisation préféré de l'invention, le mélange homogène de microbiote comprend les 15 genres bactériens suivants, producteurs de butyrate, à savoir *Blautia, Faecalibacterium, Alistipes, Eubacterium, Bifidobacterium, Ruminococcus, Clostridium, Coprococcus, Odoribacter, Roseburia, Holdemanella, Anaerostipes, Oscillibacter, Subdoligranulum* et *Butyrivibrio.*

La présence des 15 genres bactériens précités producteurs de butyrate dans le produit destiné à être administré au patient est importante car ces genres bactériens sont associés à des propriétés anti-inflammatoires. Ainsi, d'un point de vue thérapeutique, leur présence dans les mélanges homogènes est avantageuse pour l'utilisation de ces mélanges homogènes dans le traitement de l'inflammation intestinale, notamment, celle associée à la dysbiose intestinale.

Le mélange homogène peut être facilement produit d'une manière fiable et reproductible. Les résultats de l'Exemple 1 montrent une homogénéité de viabilité et de profil taxonomique entre toutes les poches de mélange produit. La qualité de l'échantillon de microbiote (le mélange homogène) administré est reproductible, et est identique entre poches issues d'un groupe de donneurs. Un produit quasi-identique peut être administré avec chaque poche. Le patient peut ainsi recevoir le même produit lors de plusieurs traitements, si plus d'un traitement est nécessaire.

En général, *n* donneurs peuvent donner suffisamment de mélange homogène pour remplir environ 3*n*, de préférence 3,2*n* poches, chaque poche étant suffisante pour un traitement de TMF.

Le mélange homogène de microbiote (ou inocula regroupés) peut être utilisé dans le traitement des dysbioses intestinales et des pathologies associées. Il en représente en effet le principe actif. La diversité bactérienne de l'inoculum regroupé, produit avec le procédé de l'invention, est élevée, pouvant avoir un indice de Shannon entre 4 et 4,50, et un inverse de l'indice de Simpson situé entre 20,2 et 27,2. Les demandeurs ont aussi démontré que les inocula individuels ont des viabilités très variées, mais que le mélange d'inoculum (inoculum regroupé) a une excellente viabilité (de 41% à 60% environ). Selon un mode de réalisation de l'invention, le mélange homogène d'inoculum a une viabilité supérieure à 40%, de préférence supérieure à 45% et, plus préférentiellement, supérieure à 50%, plus préférentiellement encore, supérieure à 55%. Les critères diversité bactérienne et viabilité sont très importants dans l'évaluation de qualité d'un produit pour TMF.

Il est aussi important que les produits soient homogènes, selon des critères règlementaires pour des produits pharmaceutiques. Les données dans les exemples ci-dessous démontrent que les produits selon l'invention sont homogènes.

Selon un mode de réalisation de l'invention, le mélange homogène de microbiote (ou inocula regroupés) peut être administré par voie rectale. Selon un mode de réalisation de l'invention, le mélange homogène de microbiote (ou inocula regroupés) peut être formulé pour administration par voie orale. Comme formulation orale, on peut mentionner les formulations mentionnées dans la demande de brevet EP 17306602.8.

Comme mentionné plus haut, des études montrent que le TMF peut être efficace dans le traitement de la maladie du greffon contre l'hôte (GvHD). Ainsi, le mélange homogène de microbiote de l'invention peut être utilisé dans le traitement de la GvHD. Par ailleurs, la présence des 15 genres bactériens précités producteurs de butyrate peut contribuer à l'efficacité du traitement de la maladie du greffon contre l'hôte (GvHD). Ainsi, selon un mode de réalisation de l'invention, un patient qui souffre de GvHD peut recevoir un TMF allogénique comprenant le mélange homogène de l'invention. Par exemple le patient peut recevoir un TMF allogénique par voie rectale. Il peut également le recevoir par voie orale.

Le mélange homogène de microbiote peut être utilisé dans le traitement de la dysbiose intestinale iatrogène et/ou des pathologies et complications associées comprenant l'inflammation intestinale et la diarrhée. Par ailleurs, la présence des 15 genres bactériens précités producteurs de butyrate ayant un effet anti-inflammatoire peut contribuer à l'efficacité du traitement.

Le mélange homogène de microbiote peut être utilisé dans le traitement de l'infection à *Clostridium difficile* et la diarrhée associée (CDI), la maladie intestinale inflammatoire (IBD), le syndrome du côlon irritable (IBS), la constipation idiopathique, la maladie coeliaque, la maladie de Crohn, l'obésité et l'obésité morbide, l'autisme, la sclérose en plaques, la diarrhée du voyageur, la maladie de Parkinson, le diabète de type II, les allergies alimentaires, le cancer, la maladie d'Alzheimer, la schizophrénie et les troubles bipolaires, la dysbiose intestinale associée à une chimiothérapie anticancéreuse ou à une immunothérapie et la maladie alcoolique et non-alcoolique du foie.

Le mélange homogène de microbiote peut être utilisé dans le traitement des complications dues à l'hospitalisation en soins intensifs.

L'invention est en outre décrite en référence aux exemples suivants. Il sera apprécié que l'invention telle que revendiquée n'est pas destinée à être limitée de quelque manière que ce soit par ces exemples.

### EXEMPLES

### Exemple 1 :

Etapes a) et b) : Huit selles fraîches de six donneurs sains ont été collectées dans le dispositif médical décrit dans WO2016/170290, puis conservées à +2°C/+ 8°C pendant une période de 72 heures, pendant laquelle des contrôles qualitatifs (étape c)) ont été réalisés. Sur la base des résultats de ces contrôles, les selles des donneurs 3 et 7 ont été rejetées.

Les selles 1, 4, 5, 6 et 2+ 8 ont été transformées séparément en un inoculum liquide en ajoutant du diluant cryoprotecteur (solution aqueuse saline contenant un mélange de maltodextrine et de tréhalose à hauteur de 20%) et clarifiées à travers un filtre (265 µm). Les selles 2 et 8 ont été combinées pour former un inoculum étant donné les faibles quantités de selles prélevées.

La viabilité des inocula 1, 4, 5, 6 et 2+ 8 (cinq inocula) a été testée. Les inocula individuels ont ensuite été transférés dans une poche souple de 5L en utilisant une pompe péristaltique. La poche a ensuite été posée sur une plaque d'agitation dans un incubateur réfrigéré réglé à 4°C et le mélange d'inoculum a été réalisé à 125 tr/min +/- 5% pendant 30 minutes.

Une fois l'homogénéisation terminée, le mélange d'inoculum a été transféré dans une série de 15 poches adaptées à une lyophilisation et conservées à -80°C. La viabilité et l'impact de la conservation dans différentes conditions du mélange homogène ont été testés pour chacune des 15 poches avant stockage.

### Analyse de viabilité :

Les tests de viabilité ont été réalisés en utilisant la technologie de cytométrie en flux utilisant un cytomètre Accuri 6 (BD Science). Les échantillons ont été dilués dans une solution aqueuse saline à 0,9%, avec dilution en série 1: 10, jusqu'à 1: 10⁻³. Les échantillons ont été marqués avec des fluorophores l'iodure de propidium (PI) (10µL/mL) et du SYTO9^{®} 9 (3µL/mL). Le PI cible également l'ADN mais pénètre uniquement dans les cellules dont les membranes sont endommagées ; il émet à 635nm (rouge) après excitation à 470nm. Le SYTO9^{®} pénètre dans toutes les cellules intègres ou non, se fixe à l'ADN et émet à 540nm (vert) après excitation à 470nm (laser bleu). Les échantillons de selles, d'inoculum ou de mélange homogène sont marqués avec le mélange des deux fluorophores avant d'être analysés par cytométrie de flux. Le pourcentage de bactéries vivantes par rapport au nombre de bactéries totales (vivantes et mortes) permet d'obtenir la viabilité bactérienne de l'échantillon. Chaque lot de l'analyse a été validé avec un témoin positif (échantillon d'inoculum de référence conservé à -80°C) et un témoin négatif (échantillon de référence conservé à -80°C et traité par incubation de 10 minutes dans 70% d'isopropanol (rapport 1/9), centrifugé, remis en suspension dans 0,9% de NaCl et dilué en séries de dilution de 10 fois jusqu'à 10⁻³).

### Résultats :

L'évaluation de la viabilité des inocula individuels varie de 30,5% à 67,6%. Les 15 poches préparées avec les mêmes mélanges d'inocula montrent une viabilité moyenne de 47,1% avec un écart-type de 1,98. Les pourcentages de viabilité du microbiote des inocula individuels (inoculum 1, 4, 5, 6 et 2 + 8, colorés en gris clair) et du mélange d'inoculum dans la poche de stockage 1 à 15 (colorés en noir) sont présentés sur la Figure 2.

Les résultats résumés sont présentés dans le Tableau 1.

**Tableau 1: Résumé de la viabilité du microbiote de l'inoculum allogénique analysé Analyse Statistique :**

| **Analyses** | **Viabilité (%)** |
|---|---|
| Moyenne | 47,1 |
| Ecart-type | 1,98 |
| Min | 44,3 |
| Max | 51,4 |

La viabilité et le nombre d'événements ont été mesurés sur les 15 poches de mélange homogène. Afin d'évaluer si ces deux mesures étaient homogènes entre les poches, une assignation aléatoire de mesures au Groupe A pour 7 poches et au Groupe B pour les poches restantes a été effectuée 500 fois.

Pour chaque itération, un test-t et un test de rang ont été utilisés pour comparer le Groupe A au Groupe B. Les résultats finaux présentés ci-dessous correspondent à la proportion d'itérations où le test statistique considéré n'était pas significatif (> 0,1). La conclusion est que les poches sont homogènes en termes de viabilité et en termes de nombre d'événements/µL.

**Tableau 2: Résultats statistiques**

| **Test** | **Viabilité** | **Nombre d'évènements/µL** |
|---|---|---|
| Proportion d'itérations où le test-t n'est pas significatif | 93,4% | 95,4% |
| Proportion d'itérations où le test de rang n'est pas significatif | 95,4% | 96,8% |

Les résultats montrent qu'il n'existe pas de différence significative pour le test-t et le test de rang, que ce soit pour la viabilité du microbiote que pour le nombre d'événements/µL.

On peut conclure que les 15 poches de mélange préparées sont homogènes entre elles.

### Conservation :

Pendant le procédé de préparation du mélange, un stockage intermédiaire doit être effectué pendant que les contrôles qualitatifs sont effectués. Afin de déterminer l'impact de ce stockage intermédiaire sur la viabilité du microbiote, une évaluation a été réalisée :
- condition de contrôle : une fois préparé, l'inoculum est instantanément conservé à -80°C.
- condition de conservation à température ambiante : une fois préparé, l'inoculum est laissé à température ambiante pendant 16 heures, un échantillonnage est effectué pour déterminer la viabilité du microbiote, les événements du microbiote/µL et la mesure du pH et de la pO2, puis l'inoculum est conservé à -80°C.
- condition de conservation à 4°C : une fois préparé, l'inoculum est conservé à 4°C pendant 16 heures, un échantillonnage est effectué pour déterminer la viabilité du microbiote, les événements du microbiote/µL et la mesure du pH et de la pO2, puis l'inoculum est conservé à -80°C.

Puis, les trois inocula sont décongelés et la viabilité du microbiote est mesurée.

Les données montrent que la médiane de viabilité du microbiote est la même pour l'inoculum conservé pendant 16 heures à température ambiante ou à 4°C. Après décongélation, la médiane de viabilité du microbiote diminue à la fois pour l'inoculum conservé à 4°C et conservé à température ambiante. Il est attendu que la viabilité baisse après congélation. Ici, on constate que dans les deux cas (4°C et température ambiante), on observe le même phénomène.

On peut observer que le nombre d'événements/µL est le même pour l'inoculum et pour l'inoculum conservé à 4°C, mais, comme on peut s'y attendre, il augmente pour l'inoculum conservé à température ambiante. La même observation est faite après décongélation de l'inoculum.

Tous ces résultats montrent que la conservation pendant 16 heures n'a pas d'impact sur la viabilité du microbiote lorsqu'il est conservé à 4°C ou à température ambiante ; le nombre d'événements/µL augmente à température ambiante indiquant que le microbiote évolue, alors qu'à 4°C le microbiote est à l'état latent. Les résultats de mesure du pH et de la pO2 sont indiqués dans le Tableau 3.

**Tableau 3: Résultats de la mesure du pH et de la pO₂**

| **Echantillon** | **PH** | **PO₂ (%)** |
|---|---|---|
| Inoculum | 6,33 | 1,4 |
| Conservation pendant 16h à température ambiante | 5,34 | 4,3 |
| Conservation pendant 16h à 4°C | 6,28 | 1,6 |
| Témoin décongélation | 6,55 | 1,5 |
| Décongélation de l'inoculum conservé à température ambiante 16h | 5,29 | 3,7 |
| Décongélation de l'inoculum conservé à 4°C pendant 16h | 6,23 | 1,3 |

Ces résultats montrent que la conservation pendant 16 heures n'a pas d'impact sur le pH et sur la pO2 de l'inoculum conservé à 4°C.

### Exemple 2 :

En utilisant presque les mêmes conditions que pour l'Exemple 1, le procédé a été réalisé sur quatre lots (lot N°1 à lot N°4) d'échantillons de microbiote fécal, avec 2, 8, 4 et 6 donneurs pour le lot N°1, le lot N°2, le lot N°3 et le lot N° 4 respectivement. Le lot N° 4 correspond au lot de l'Exemple 1. Ainsi, pour le lot N°4, deux des six selles ont été combinées pour former un inoculum étant donné les faibles quantités de selles prélevées.

Les selles ont été transformées séparément en un inoculum liquide en ajoutant du diluant cryoprotecteur (solution aqueuse saline contenant un mélange de maltodextrine et de tréhalose à hauteur de 20%) et clarifiées à travers un filtre (265 µm).

La viabilité et le profil taxonomique des inocula ont été testés. Les inocula individuels ont ensuite été transférés dans une poche souple de 3L ou 5L en utilisant une pompe péristaltique. La poche a ensuite été posée sur une plaque d'agitation dans un incubateur réfrigéré réglé à 4°C et le mélange d'inoculum a été réalisé à 130 tr/min +/-5% pendant 30 minutes dans un incubateur réglé à 4°C.

Une fois l'homogénéisation terminée, pour chaque lot, le mélange d'inoculum a été transféré dans une série de poches adaptées à une congélation et conservées à -80°C. La viabilité et le profil taxonomique des mélanges homogènes ont été testés avant stockage. Pour le lot N°1, 2 selles ont été collectées et 5 poches ont été remplies. Pour le lot N°2, 8 selles ont été collectées et 29 poches ont été remplies. Pour le lot N°3, 5 selles ont été collectées et 21 poches ont été remplies. Pour le lot N°4, 6 selles ont été collectées, 2 selles ont été combinées pour avoir assez de matière pour former un inoculum et 15 poches ont été remplies.

### Analyse de viabilité :

Les tests de viabilité ont été réalisés comme pour l'Exemple 1.

### Analyse métagénomique :

L'ADN génomique a été extrait des échantillons avec le kit NucleoSpin Soil (Machery Nagel). Une banque de séquençage a été construite pour chaque échantillon avec le kit MyTaq HS-Mix (Bioline). Les banques ont ensuite été séquencées sur un run MiSeq V3 2x300 pb.

Après un contrôle qualité des séquences démultiplexées avec Trimmomatic [Bolger et al., (2014) 'Trimmomatic: A flexible trimmer for Illumina sequence data', Bioinformatics, 30(15), pp. 2114-2120. doi: 10.1093/bioinformatics/btu170], les séquences humaines ont été éliminées en utilisant le logiciel Bowtie2 (Langmead, Ben and Salzberg, (2013) 'Fast gapped-read alignment with Bowtie2', Nature methods, 9(4), pp. 357-359. doi: 10.1038/nmeth.1923.Fast). Pour permettre la comparaison des données, le nombre de séquences a été normalisé à 60 000 séquences par échantillon. Le clustering des séquences a été réalisé avec VSEARCH et l'assignation taxonomique a ensuite été réalisée en utilisant la base de données Silva 128. Les analyses taxonomiques et les mesures de diversité ont été réalisées avec le logiciel R (R Core Team 2015, version 3.4.4, http://www.R-project.org). Pour les mesures de diversité, 20 sous-échantillonnages de 60 000 séquences ont été réalisés pour chaque échantillon et la médiane a été mesurée.

### Résultats :

### Viabilité :

Le **Tableau 4,** ci-dessous, montre la viabilité des lots. L'évaluation de la viabilité des inocula individuels varie de 16,8% à 67,6%. La viabilité des lots des inocula regroupés est donnée dans le Tableau ci-dessous, et se situe entre 46,1 et 60,2%.

**Tableau 4**

| **Analyses** | **Lot N°1** | **Lot N°2** | **Lot N°3** | **Lot N°4** |
|---|---|---|---|---|
| Nombre de donneurs | 2 | 7 | 4 | 6 |
| Moyenne (%) | 60,2 | 46,1 | 53,2 | 47,1 |
| Ecart-type | 1,2 | 1,1 | 2,1 | 2,0 |
| Min | 59,4 | 45,0 | 50,6 | 44,3 |
| Max | 61,6 | 48,3 | 58,2 | 51,4 |

Le **Tableau 5** ci-dessous montre les résultats.

**Tableau 5**

| **Analyses** | | **Inverse de l'indice de Simpson** | **Shannon** | **Protéobactérie** |
|---|---|---|---|---|
| Lot N°1 | Donneur 1 | 17,68 | 3,8 | |
| | Donneur 2 | 8,23 | 2,6 | |
| | **Mélange d'inocula** | 20,2 | 4 | 5,6 |
| Lot N°2 | Donneur 1 | 17,4 | 3,9 | |
| | Donneur 2 | 7,5 | 3 | |
| | Donneur 3 | 16,4 | 3,5 | |
| | Donneur 4 | 16,3 | 3,48 | |
| | Donneur 5 | 26 | 3,8 | |
| | Donneur 6 | 12,4 | 3,4 | |
| | Donneur 7 | 22,07 | 4 | |
| | **Mélange d'inocula** | 26,2 | 4,3 | 3,5 |
| Lot N°3 | Donneur 1 | 21,06 | 3,98 | |
| | Donneur 2 | 29,15 | 4,1 | |
| | Donneur 3 | 20,28 | 3,7 | |
| | Donneur 4 | 6,5 | 2,5 | |
| | **Mélange d'inocula** | 27,2 | 4,3 | 3,7 |
| Lot N°4 | Donneur 1 | 12,3 | 3,59 | |
| | Donneur 4 | 16,37 | 4,1 | |
| | Donneur 5 | 21,37 | 3,48 | |
| | Donneur 6 | 16,71 | 4 | |
| | Donneur 2+ Donneur 8 | 25,12 | 3,52 | |
| | **Mélange d'inocula** | 26,4 | 3,27 | 3,1 |

### Exemple 3 :

Cette étude a pour objectif de comparer deux méthodes de préparation d'échantillons de microbiote fécal de donneurs multiples :
- la méthode décrite dans l'Exemple 2 et
- la méthode de l'art antérieur de Paramsothy et al. [Paramsothy S., et al. (2017), Multidonor intensive faecal microbiota transplantation for active ulcerative colitis: a randomised placebo-controlled trial, Lancet, Mar 25; 389(10075): 1218-1228]. La figure 5 montre les deux méthodes utilisées.

Des analyses de viabilité et des analyses métagénomiques ont été réalisées afin de comparer la viabilité des produits finaux obtenus, ainsi que leur homogénéité taxonomique.

Quatre selles fraîches de quatre donneurs sains ont chacune été collectées dans le dispositif médical décrit dans WO2016/170290, puis conservées à +2°C/+ 8°C pendant une période de 72 heures, pendant laquelle des contrôles qualitatifs (étape c)) ont été réalisés.

Les quatre selles ont été divisées en deux fractions, l'une destinée au traitement selon un mode de réalisation de l'invention (« Inv ») et l'autre selon la méthode de l'art antérieur de Paramsothy et al. (« Ref »).

Les selles « Inv » ont été transformées séparément en un inoculum liquide en ajoutant du diluant cryoprotecteur (solution aqueuse saline contenant un mélange de maltodextrine et de tréhalose à hauteur de 20%) et clarifiées à travers un filtre (265 µm).

Les inocula individuels ont ensuite été transférés dans une poche souple de 1L en utilisant une pompe péristaltique. La poche a ensuite été posée sur une plaque d'agitation dans un incubateur réfrigéré réglé à 4°C et le mélange d'inoculum a été réalisé à 125 tr/min +/- 5% pendant 30 minutes.

Une fois l'homogénéisation terminée, le mélange d'inoculum a été transféré dans une série de 10 cryotubes et conservé à -80°C.

Les selles « Ref » ont été mélangées de sorte à obtenir un produit visuellement homogène, clarifiées dans une solution saline isotonique (0,9% de NaCl), puis filtrées. 10% de glycérol ont ensuite été ajoutés à la préparation pour le stockage. Puis, l'échantillon ainsi produit a été transféré dans une série de 10 cryotubes et conservé à - 80°C.

La viabilité et l'analyse métagénomique 16S ont été réalisées comme décrit dans l'Exemple 2. Les résultats sont commentés dans le texte ci-dessous.

Le Tableau 6 ci-dessous montre l'abondance relative de Actinobacteria, *Prevotella* et *Bifidobacteria* dans les échantillons « Inv » et « Ref ».

**Tableau 6**

| | **Abondance Relative** | | **Coefficient de Variation** | | |
|---|---|---|---|---|---|
| | Inv | Réf | Inv | Réf | Test de Rang Wilcoxon |
| Actinobacteria | 3,6% | 0,9% | 7,8% | 14,5% | 1,083^{e}-5 |
| Prevotella | 13% | 11% | 3,3% | 8,5% | 2,057^{e}-4 |
| Bifidobacteria | 0,9% | 0,6% | 12,4% | 13,9% | 1,083^{e}-5 |

### Exemple 4 :

Une campagne de production visant à obtenir des mélanges homogènes de selles a été réalisée comme décrit dans l'Exemple 1. Les selles de 8 donneurs répondant aux critères de sélection de l'étape c) ont été collectées de façon quotidienne sur une période de 5 semaines. Les selles ont été transformées séparément en un inoculum liquide en ajoutant du diluant cryoprotecteur (solution aqueuse saline contenant un mélange de maltodextrine et de tréhalose à hauteur de 20%), et clarifiées à travers un filtre (265 µm).

Les inocula individuels obtenus un même jour ont ensuite été mélangés de sorte à obtenir des lots de produits homogènes.

### Extraction d'ADN, séquençage métagénomique et analyse bio-informatique

L'ADN génomique a été extrait des mélanges de selles avec le kit Nucleospin Soil Kit (Macherey Nagel). Une banque de séquençage a été construite pour chaque échantillon d'ADN génomique en utilisant le kit TruSeq (Illumina) selon les recommandations du fournisseur. Les banques ont ensuite été séquencées simultanément en « paired-end » sur un run HiSeq2500 (Illumina).

Après un contrôle qualité des séquences démultiplexées avec Trimmomatic [Bolger et al., (2014) 'Trimmomatic: A flexible trimmer for Illumina sequence data', Bioinformatics, 30(15), pp. 2114-2120. doi: 10.1093/bioinformatics/btu170], les séquences humaines ont été éliminées en utilisant le logiciel Bowtie2 (Langmead, Ben and Salzberg, (2013) 'Fast gapped-read alignment with Bowtie2', Nature methods, 9(4), pp. 357-359. doi: 10.1038/nmeth.1923.Fast). Pour permettre la comparaison des données, le nombre de séquences a été normalisé à 20 000 000 séquences « paired-end » par échantillon. L'assignation taxonomique a ensuite été réalisée avec Kraken v.0.10.5-beta (Wood and Salzberg, 2014 'Kraken: ultrafast metagenomic sequence classification using exact alignments', Genome Biology, 15(3), pp. R46. doi: 10.1186/gb-2014-15-3-r46) en utilisant la base de données RefSeq genomic (Septembre 2017, http://www.ncbi.nlm.nih.gov/refseq/).

### Résultats :

La Figure 8 montre l'abondance relative des 15 genres bactériens producteurs de butyrate, à savoir *Blautia, Faecalibacterium, Alistipes, Eubacterium, Bifidobacterium, Ruminococcus, Clostridium, Coprococcus, Odoribacter, Roseburia, Holdemanella, Anaerostipes, Oscillibacter, Subdoligranulum et Butyrivibrio,* dans les selles des donneurs (première barre à gauche), puis dans les lots des mélanges homogènes obtenus à partir des selles de 3, 4, 5 et 6 donneurs respectivement. L'abondance relative de ces genres est stable dans chaque lot et se standardise quand le nombre de selles utilisées pour obtenir le mélange homogène augmente (voir Tableau 7 ci-dessous).

**Tableau 7 : Coefficients de variation de 15 genres bactériens producteurs de butyrate chez les donneurs et dans les différents lots**

| **Donneurs** | **Lots de 3 selles** | **Lots de 4 selles** | **Lots de 5 selles** | **Lots de 6 selles** |
|---|---|---|---|---|
| 0,376 | 0,196 | 0,093 | 0,086 | 0,039 |

Si les 15 genres bactériens producteurs de butyrate ne se retrouvent pas tous dans toutes les selles individuelles, en revanche, les lots fabriqués avec un mélange d'au moins quatre selles les contiennent tous (données non montrées).

## Revendications

1. Procédé de préparation d'un mélange homogène de microbiote fécal provenant d'au moins deux donneurs présélectionnés comprenant les étapes suivantes :
a. le prélèvement d'au moins un échantillon de microbiote fécal desdits donneurs présélectionnés,
b. dans un délai inférieur à 5 minutes suivant le prélèvement, le placement de l'échantillon obtenu en a) dans un dispositif de collecte étanche à l'oxygène,
c. le contrôle qualitatif des échantillons prélevés et l'exclusion des échantillons ne répondant pas aux critères qualitatifs,
d. l'ajout à chacun des échantillons retenus après l'étape de contrôle c) d'une solution aqueuse saline comprenant au moins un agent cryoprotecteur et/ou un agent de charge,
e. la filtration des échantillons obtenus à l'issue de l'étape d) pour former une série d'inocula,
f. le regroupement desdits inocula pour former un mélange d'inocula,
g. l'homogénéisation dudit mélange obtenu à l'étape f), notamment par agitation manuelle ou à l'aide d'un dispositif d'agitation, les étapes b) et d) à g) étant réalisées en anaérobiose.

2. Procédé selon la revendication 1, **caractérisé en ce que** le mélange homogène de microbiote fécal provient d'au moins quatre donneurs.

3. Procédé selon la revendication 1 ou 2, **caractérisé en ce que** les donneurs sont présélectionnés selon les critères de présélection suivants :
i. ayant entre 18 et 60 ans,
ii. ayant un Indice de Masse Corporel (IMC) entre 18 et 30,
iii. absence d'antécédents personnels de maladies infectieuses graves, de troubles métaboliques et neurologiques, ou de dépression,
iv. absence de prise récente de médicaments pouvant détériorer la composition du microbiote intestinal,
v. absence d'apparition récente de symptômes associés à une maladie gastro intestinale, tels que fièvre, diarrhée, nausée, vomissement, douleur abdominale, jaunisse, absence d'historique de maladies infectieuses graves, notamment le SIDA, l'hépatite etc.
vi. absence de voyages récents dans des pays tropicaux
vii. absence de comportement sexuel à risque,
viii. absence de blessure, piercing et/ou tatouage récent(s),
ix. absence de fatigue chronique récente,
x. absence de réaction allergique récente,
xi. optionnellement ayant un régime alimentaire divers.

4. Procédé selon la revendication 1,2 ou 3, **caractérisé en ce que** les critères qualitatifs des échantillons de l'étape c) comprennent :
- consistance de l'échantillon entre 1 et 6 selon l'échelle de Bristol,
- absence de sang et d'urine dans l'échantillon,

5. Procédé selon la revendication 4, **caractérisé en ce que** les critères qualitatifs des échantillons de l'étape c) comprennent :
- absence des bactéries suivantes : *Campylobacter, Clostridium difficile (toxin A*/*B), Salmonella Yersinia enterocolitica, Vibrio sp., Shiga-like toxin-producing E.coli (STEC) stxl*/*stx2,* des bactéries multi résistantes, bactéries productrices de Bêta-lactamase à spectre étendu (BLSE) - Entérocoques résistant à la vancomycine et aux glycopeptides (ERV, GRE), *Listeria monocytogenes,* et les *bactéries résistantes aux carbapénèmes,*
- absence des parasites suivants : *Cryptosporidium parvum,* Cyclospora sp., *Entamoeba histolytica, Giardia lamblia, Blastocystis hominis, Helminths, Strongyloides stercoralis, Isospora sp., Microsporidies et Dientamoeba fragilis,*
- absence des virus suivants : Adenovirus F40/41, Astrovirus, Norovirus, Rotavirus A, Sapovirus et Picornavirus (Aichi Virus et enterovirus),
- absence des bactéries suivantes : *E.coli enteroaggregative* (EAEC), E*.coli enteropathogenic* (EPEC), E*.coli enterotoxigenic* (ETEC) It/st, *Shigella*/*Enteroinvasive E.coli* (EIEC) et *Plesiomonas shigelloides.*

6. Procédé selon la revendication 4, **caractérisé en ce que** les critères qualitatifs des échantillons de l'étape c) comprennent :
- absence des bactéries suivantes : *Campylobacter, Clostridium difficile (toxin A*/*B), Salmonella Yersinia enterocolitica, Vibrio sp., Shiga-like toxin-producing E.coli (STEC) stxl*/*stx2,* des bactéries multi résistantes, bactéries productrices de Bêta-lactamase à spectre étendu (BLSE) - Entérocoques résistant à la vancomycine et aux glycopeptides (ERV, GRE) et *Listeria monocytogenes,*
- absence des parasites suivants : *Cryptosporidium parvum,* Cyclospora sp., *Entamoeba histolytica, Giardia lamblia, Blastocystis hominis, Helminths, Strongyloides stercoralis, Isospora sp., Microsporidies et Dientamoeba fragilis,*
- absence des virus suivants : Adenovirus F40/41, Astrovirus, Norovirus, Rotavirus A, Sapovirus et Picornavirus (Aichi Virus et enterovirus),
- absence des bactéries suivantes : *E.coli enteroaggregative* (EAEC), E*.coli enteropathogenic* (EPEC), E*.coli enterotoxigenic* (ETEC) It/st, *Shigella*/*Enteroinvasive E.coli* (EIEC) et *Plesiomonas shigelloides.*

7. Procédé selon l'une des revendications 1 à 6, **caractérisé en ce que** l'au moins un agent cryoprotecteur et/ou agent de charge est un polyol, un di-, tri- ou polysaccaride ou leur mélange et un agent de charge.

8. Procédé selon l'une des revendications 1 à 7, **caractérisé en ce que** la solution aqueuse saline comprend maltodextrine et tréhalose.

9. Procédé selon l'une des revendications 1 à 8, **caractérisé en ce que** la filtration à l'étape e) est réalisée avec un filtre comprenant des pores de diamètre inférieur ou égal à 0,5 mm, de préférence, inférieur ou égal à 265 µm.

10. Procédé selon l'une des revendications 1 à 9, **caractérisé en ce que** le temps entre la collecte de l'échantillon et la fin de l'étape g) est moins de 76 heures.

11. Procédé selon l'une des revendications 1 à 10, **caractérisé en ce que** l'étape g) d'homogénéisation est réalisée à une température entre 2°C et 25°C, de préférence entre environ 2 et 6°C, plus préférentiellement à environ 4°C.

12. Procédé selon l'une des revendications 1 à 11, **caractérisé en ce qu'**il comprend l'étape de transfert suivante :
h. le transfert du mélange homogénéisé obtenu à l'étape g) :
i. dans des poches d'inoculum pour stockage à une température d'environ -50°C à -80°C, de préférence, à -80°C, ou pour stockage à une température entre environ 2 à 6 °C pour utilisation du mélange sous 16 heures environ, ou pour stockage à une température entre 10 à 25 °C pour utilisation du mélange sous 4 heures environ,
ii. dans un dispositif de lyophilisation pour lyophilisation.

13. Procédé selon l'une des revendications 2 à 12, **caractérisé en ce que** le mélange homogène de microbiote fécal contient 15 genres bactériens *Blautia, Faecalibacterium, Alistipes, Eubacterium, Bifidobacterium, Ruminococcus, Clostridium, Coprococcus, Odoribacter, Roseburia, Holdemanella, Anaerostipes, Oscillibacter, Subdoligranulum* et *Butyrivibrio.*

14. Mélange homogène de microbiote fécal susceptible d'être obtenu selon l'une des revendications 1 à 13 pour son utilisation dans le traitement des dysbioses intestinales et/ou des maladies associées aux dysbioses intestinales par transplantation de microbiote fécal allogénique.

15. Mélange homogène de microbiote fécal susceptible d'être obtenu selon l'une des revendications 1 à 13 **caractérisé en ce que** le mélange a un inverse de l'indice de Simpson supérieur à 15, de préférence 20.

16. Mélange homogène de microbiote fécal susceptible d'être obtenu selon l'une des revendications 1 à 13 pour son utilisation dans le traitement des dysbioses intestinales.

17. Mélange homogène de microbiote fécal susceptible d'être obtenu selon l'une des revendications 1 à 13 pour son utilisation dans le traitement de la maladie du greffon contre l'hôte (GvHD).

18. Mélange homogène de microbiote fécal susceptible d'être obtenu selon l'une des revendications 1 à 13 pour son utilisation dans le traitement de la dysbiose intestinale iatrogène et/ou des pathologies et complications associées comprenant l'inflammation intestinale, la diarrhée.

19. Mélange homogène de microbiote fécal susceptible d'être obtenu selon l'une des revendications 1 à 13 pour son utilisation pour le traitement de l'infection à *Clostridium difficile* et la diarrhée associée (CDI), la maladie intestinale inflammatoire (IBD), le syndrome du côlon irritable (IBS), la constipation idiopathique, la maladie coeliaque, la maladie de Crohn, l'obésité et l'obésité morbide, l'autisme, la sclérose en plaques, la diarrhée du voyageur, la maladie de Parkinson, le diabète de type II, les allergies alimentaires, le cancer, la maladie d'Alzheimer, la schizophrénie et les troubles bipolaires, la dysbiose intestinale associée à une chimiothérapie anticancéreuse ou à une immunothérapie et la maladie alcoolique et non-alcoolique du foie.

## Patentansprüche

1. Verfahren zum Herstellen einer homogenen Mischung fäkaler Mikrobiota, die von mindestens zwei vorab ausgewählten Spendern stammt, umfassend die folgenden Schritte:
a. Entnehmen mindestens einer Probe fäkaler Mikrobiota von den vorab ausgewählten Spendern,
b. Einbringen der in a) gewonnenen Probe in eine sauerstoffdichte Sammelvorrichtung innerhalb eines Zeitraums von weniger als 5 Minuten nach der Entnahme,
c. Kontrollieren der Qualität der entnommenen Proben und Ausschließen der Proben, die die Qualitätskriterien nicht erfüllen,
d. Zugeben einer wässrigen Kochsalzlösung, die mindestens ein Kälteschutzmittel und/oder einen Füllstoff umfasst, zu jeder der nach dem Kontrollschritt c) übernommenen Proben,
e. Filtern der im Anschluss an Schritt d) erhaltenen Proben, um eine Reihe von Inokula zu bilden,
f. Gruppieren der Inokula, um eine Mischung aus Inokula zu bilden, und
g. Homogenisieren der in Schritt f) erhaltenen Mischung, insbesondere durch manuelles Rühren oder mit Hilfe einer Rührvorrichtung,
wobei die Schritte b) und d) bis g) unter anaeroben Bedingungen durchgeführt werden.

2. Verfahren nach Anspruch 1, **dadurch gekennzeichnet, dass** die homogene Mischung fäkaler Mikrobiota von mindestens vier Spendern stammt.

3. Verfahren nach Anspruch 1 oder 2, **dadurch gekennzeichnet, dass** die Spender gemäß den folgenden Vorauswahlkriterien vorab ausgewählt werden:
i. Alter zwischen 18 und 60 Jahren,
ii. Body-Mass-Index (BMI) zwischen 18 und 30,
iii. Fehlen einer persönlichen Krankengeschichte mit schwerwiegenden Infektionskrankheiten, Stoffwechsel- und neurologischen Störungen oder Depressionen,
iv. Fehlen kürzlich eingenommener Medikamente, die die Zusammensetzung der Darmmikrobiota beeinträchtigen könnten,
v. Fehlen eines kürzlichen Auftretens von Symptomen im Zusammenhang mit einer Magen-Darm-Erkrankung, wie Fieber, Durchfall, Übelkeit, Erbrechen, Magenschmerzen, Gelbsucht, Fehlen einer Geschichte von schweren Infektionskrankheiten, insbesondere AIDS, Hepatitis usw.
vi. Fehlen von kürzlichen Reisen in tropische Länder,
vii. Fehlen von riskantem Sexualverhalten,
viii. Fehlen frischer Wunden, Piercings und/oder Tätowierungen,
ix. Fehlen einer kürzlich aufgetretenen chronischen Müdigkeit,
x. Fehlen einer kürzlich aufgetretenen allergischen Reaktion,
xi. optional Vorhandensein einer unterschiedlichen Ernährungsweise.

4. Verfahren nach Anspruch 1, 2 oder 3, **dadurch gekennzeichnet, dass** die Qualitätskriterien der Proben von Schritt c) Folgendes umfassen:
- Konsistenz der Probe zwischen 1 und 6 gemäß der Bristol-Skala,
- Fehlen von Blut oder Urin in der Probe.

5. Verfahren nach Anspruch 4, **dadurch gekennzeichnet, dass** die Qualitätskriterien der Proben von Schritt c) Folgendes umfassen:
- Fehlen der folgenden Bakterien: *Campylobacter, Clostridium difficile* (A/B-Toxin), *Salmonella Yersinia enterocolitica, Vibrio sp., Shigatoxin-bildende E. coli (STEC) stx1*/*stx2,* multiresistente Bakterien, Bakterien, die Beta-Laktamasen mit erweitertem Spektrum (ESBL) bilden - Vancomycin- und Glykopeptid-resistente Enterokokken (VRE, GRE), *Listeria monocytogenes* und *Carbapenem-resistente Bakterien,*
- Fehlen der folgenden Parasiten: *Cryptosporidium parvum,* Cyclospora sp., *Entamoeba histolytica, Giardia lamblia, Blastocystis hominis, Helminthen, Strongyloides stercoralis, Isospora sp., Microsporidia und Dientamoeba fragilis,*
- Fehlen der folgenden Viren: Adenovirus F40/41, Astrovirus, Norovirus, Rotavirus A, Sapovirus und Picornavirus (Aichi-Virus und Enterovirus),
- Fehlen der folgenden Bakterien: *enteroaggregative E. coli* (EAEC), *enteropathogene E. coli* (EPEC), *enterotoxische E. coli* (ETEC) It/st, *Shigella*/*enteroinvasive E. coli* (EIEC) und *Plesiomonas shigelloides.*

6. Verfahren nach Anspruch 4, **dadurch gekennzeichnet, dass** die Qualitätskriterien der Proben von Schritt c) Folgendes umfassen:
- Fehlen der folgenden Bakterien: *Campylobacter, Clostridium difficile* (A/B-Toxin), *Salmonella Yersinia enterocolitica, Vibrio sp., Shigatoxin-bildende E. coli (STEC) stxl*/*stx2,* multiresistente Bakterien, Bakterien, die Beta-Laktamasen mit erweitertem Spektrum (ESBL) bilden - Vancomycin- und Glykopeptid-resistente Enterokokken (VRE, GRE) und *Listeria monocytogenes,*
- Fehlen der folgenden Parasiten: *Cryptosporidium parvum,* Cyclospora sp., *Entamoeba histolytica, Giardia lamblia, Blastocystis hominis, Helminthen, Strongyloides stercoralis, Isospora sp., Microsporidia und Dientamoeba fragilis,*
- Fehlen der folgenden Viren: Adenovirus F40/41, Astrovirus, Norovirus, Rotavirus A, Sapovirus und Picornavirus (Aichi-Virus und Enterovirus),
- Fehlen der folgenden Bakterien: *enteroaggregative E. coli* (EAEC), *enteropathogene E. coli* (EPEC), *enterotoxische E. coli* (ETEC) It/st, *Shigella*/*enteroinvasive E. coli* (EIEC) und *Plesiomonas shigelloides.*

7. Verfahren nach einem der Ansprüche 1 bis 6, **dadurch gekennzeichnet, dass** das mindestens eine Kälteschutzmittel und/oder der mindestens eine Füllstoff ein Polyol, ein Di-, Tri- oder Polysaccharid oder eine Mischung daraus und ein Füllstoff ist.

8. Verfahren nach einem der Ansprüche 1 bis 7, **dadurch gekennzeichnet, dass** die wässrige Kochsalzlösung Maltodextrin und Trehalose umfasst.

9. Verfahren nach einem der Ansprüche 1 bis 8, **dadurch gekennzeichnet, dass** das Filtern in Schritt e) mit einem Filter durchgeführt wird, der Poren mit einem Durchmesser von weniger als oder gleich 0,5 mm, vorzugsweise weniger als oder gleich 265 µm, umfasst.

10. Verfahren nach einem der Ansprüche 1 bis 9, **dadurch gekennzeichnet, dass** die Zeit zwischen dem Sammeln der Probe und dem Ende des Schritts g) weniger als 76 Stunden beträgt.

11. Verfahren nach einem der Ansprüche 1 bis 10, **dadurch gekennzeichnet, dass** der Schritt g) des Homogenisierens bei einer Temperatur zwischen 2 °C und 25 °C, vorzugsweise zwischen etwa 2 und 6 °C, noch bevorzugter bei etwa 4 °C, durchgeführt wird.

12. Verfahren nach einem der Ansprüche 1 bis 11, **dadurch gekennzeichnet, dass** es den folgenden Übertragungsschritt umfasst:
h. Übertragen der in Schritt g) erhaltenen homogenisierten Mischung:
i. in Inokulumbeuteln zur Lagerung bei einer Temperatur von etwa -50 °C bis -80 °C, vorzugsweise bei -80 °C, oder zur Lagerung bei einer Temperatur zwischen etwa 2 und 6 °C zur Verwendung der Mischung innerhalb von etwa 16 Stunden oder zur Lagerung bei einer Temperatur zwischen 10 und 25 °C zur Verwendung der Mischung innerhalb von etwa 4 Stunden,
ii. in einer Lyophilisierungsvorrichtung zur Lyophilisierung.

13. Verfahren nach einem der Ansprüche 2 bis 12, **dadurch gekennzeichnet, dass** die homogene Mischung fäkaler Mikrobiota 15 Bakteriengattungen, *Blautia, Faecalibacterium, Alistipes, Eubacterium, Bifidobacterium, Ruminococcus, Clostridium, Coprococcus, Odoribacter, Roseburia, Holdemanella, Anaerostipes, Oscillibacter, Subdoligranulum* und *Butyrivibrio,* enthält.

14. Homogene Mischung fäkaler Mikrobiota, die nach einem der Ansprüche 1 bis 13 erhalten werden kann, zur Verwendung bei der Behandlung von Darmdysbiosen und/oder Krankheiten im Zusammenhang mit Darmdysbiosen durch Transplantation allogener fäkaler Mikrobiota.

15. Homogene Mischung fäkaler Mikrobiota, die nach einem der Ansprüche 1 bis 13 erhalten werden kann, **dadurch gekennzeichnet, dass** die Mischung einen inversen Simpson-Index von mehr als 15, vorzugsweise von 20, aufweist.

16. Homogene Mischung fäkaler Mikrobiota, die nach einem der Ansprüche 1 bis 13 erhalten werden kann, zur Verwendung bei der Behandlung von Darmdysbiosen.

17. Homogene Mischung fäkaler Mikrobiota, die nach einem der Ansprüche 1 bis 13 erhalten werden kann, zur Verwendung bei der Behandlung der Graft-versus-Host-Erkrankung (GvHD).

18. Homogene Mischung fäkaler Mikrobiota, die nach einem der Ansprüche 1 bis 13 erhalten werden kann, zur Verwendung bei der Behandlung von iatrogener Darmdysbiose und/oder damit verbundenen Pathologien und Komplikationen, zu denen Darmentzündung und Durchfall zählen.

19. Homogene Mischung fäkaler Mikrobiota, die nach einem der Ansprüche 1 bis 13 erhalten werden kann, zur Verwendung bei der Behandlung von *Clostridium difficile-*Infektionen und damit verbundenem Durchfall (CDI), entzündlichen Darmerkrankungen (IBD), Reizdarmsyndrom (IBS), idiopathischer Verstopfung, Zöliakie, Morbus Crohn, Fettleibigkeit und krankhafter Fettleibigkeit, Autismus, Multipler Sklerose, Reisekrankheit, Parkinson-Krankheit, Typ-II-Diabetes, Nahrungsmittelallergien, Krebs, Alzheimer-Krankheit, Schizophrenie und bipolare Störungen, Darmdysbiose im Zusammenhang mit Krebs-Chemotherapie oder Immuntherapie sowie alkoholische oder nicht-alkoholische Lebererkrankungen.

## Claims

1. Method for the preparation of a homogenous mixture of fecal microbiota originating from at least two preselected donors comprising the following steps:
a. taking at least one sample of fecal microbiota from said preselected donors,
b. within a time period of less than 5 minutes after taking the sample, placing the sample obtained in a) in an oxygen-impermeable collection device,
c. quality control of the samples taken, and exclusion of samples that do not meet the quality criteria,
d. to each of the samples retained after the control step c), adding an aqueous saline solution comprising at least one cryoprotectant and/or a bulking agent,
e. filtering the samples obtained at the end of step d) to form a series of inocula,
f. grouping said inocula to form a mixture of inocula,
g. homogenization of said mixture obtained in step f), in particular by manual stirring or using a stirring device,
steps b) and d) to g) being carried out under anaerobiosis.

2. Method according to claim 1, **characterized in that** the homogenous mixture of fecal microbiota originates from at least four donors.

3. Method according to claim 1 or 2, **characterized in that** the donors are preselected according to the following preselection criteria:
i. age between 18 and 60 years,
ii. body mass index (BMI) between 18 and 30,
iii. absence of personal medical history of serious infectious diseases, metabolic and neurological disorders, or depression,
iv. absence of recently taking medicaments capable of degrading the composition of the intestinal microbiota,
v. absence of recent occurrence of symptoms associated with a gastrointestinal disease, such as fever, diarrhea, nausea, vomiting, abdominal pain, jaundice; absence of any history of serious infectious diseases, in particular AIDS, hepatitis, etc.
vi. absence of recent travel in tropical countries,
vii. absence of at-risk sexual behavior,
viii. absence of recent wound, piercing and/or tattoo,
ix. absence of recent chronic fatigue,
x. absence of recent allergic reaction,
xi. optionally, having a varied diet.

4. Method according to claim 1, 2 or 3, **characterized in that** the quality criteria of the samples of step c) comprise:
- consistency of the sample between 1 and 6 on the Bristol scale,
- absence of blood or urine in the sample.

5. Method according to claim 4, **characterized in that** the quality criteria of the samples of step c) comprise:
- absence of the following bacteria: *Campylobacter, Clostridium difficile (A*/*B toxin), Salmonella Yersinia enterocolitica, Vibrio sp., Shiga toxin-producing E.coli (STEC) stxl*/*stx2,* multi-resistant bacteria, bacteria producing extended-spectrum beta-lactamases (ESBL) - glycopeptide/vancomycin resistant Enterococci (GRE/VRE), *Listeria monocytogenes, and carbapenem-resistant bacteria,*
- absence of the following parasites: *Cryptosporidium parvum, Cyclospora* sp., *Entamoeba histolytica, Giardia lamblia, Blastocystis hominis, Helminths, Strongyloides stercoralis, Isospora sp., Microsporidia* and *Dientamoeba fragilis,*
- absence of the following viruses: adenovirus F40/41, astrovirus, norovirus, rotavirus A, sapovirus and picornavirus (aichi virus and enterovirus),
- absence of the following bacteria: *enteroaggregative E.coli* (EAEC), *enteropathogenic E.coli* (EPEC), *enterotoxigenic E.coli* (ETEC) It/st, *Shigella*/*enteroinvasive E.coli* (EIEC) and *Plesiomonas shigelloides.*

6. Method according to claim 4, **characterized in that** the quality criteria of the samples of step c) comprise:
- absence of the following bacteria: *Campylobacter, Clostridium difficile (A*/*B toxin), Salmonella Yersinia enterocolitica, Vibrio sp., Shiga toxin-producing E.coli (STEC) stxl*/*stx2,* multi-resistant bacteria, bacteria producing extended-spectrum beta-lactamases (ESBL) - glycopeptide/vancomycin resistant Enterococci (GRE/VRE), and *Listeria monocytogenes,*
- absence of the following parasites: *Cryptosporidium parvum, Cyclospora* sp., *Entamoeba histolytica, Giardia lamblia, Blastocystis hominis, Helminths, Strongyloides stercoralis, Isospora sp., Microsporidia* and *Dientamoeba fragilis,*
- absence of the following viruses: adenovirus F40/41, astrovirus, norovirus, rotavirus A, sapovirus and picornavirus (aichi virus and enterovirus),
- absence of the following bacteria: *enteroaggregative E.coli* (EAEC), *enteropathogenic E.coli* (EPEC), *enterotoxigenic E.coli* (ETEC) It/st, *Shigella*/*enteroinvasive E.coli* (EIEC) and *Plesiomonas shigelloides.*

7. Method according to one of claims 1 to 6, **characterized in that** the at least one cryoprotectant and/or bulking agent is a polyol, a di-, tri- or polysaccharide or mixture thereof and a bulking agent.

8. Method according to one of claims 1 to 7, **characterized in that** the aqueous saline solution comprises maltodextrin and trehalose.

9. Method according to one of claims 1 to 8, **characterized in that** the filtration in step e) is carried out with a filter comprising pores of diameter less than or equal to 0.5 mm, preferably less than or equal to 265 µm.

10. Method according to one of claims 1 to 9, **characterized in that** the time between collection of the sample and the end of step g) is less than 76 hours.

11. Method according to one of claims 1 to 10, **characterized in that** step g) of homogenization is carried out at a temperature between 2°C and 25°C, preferably between approximately 2 and 6°C, more preferentially at approximately 4°C.

12. Method according to one of claims 1 to 11, **characterized in that** it comprises the following transfer step:
h. transferring the homogenized mixture obtained in step g):
i. to inoculum pouches for storage at a temperature of approximately -50°C to -80°C, preferably, at -80°C, or for storage at a temperature between approximately 2 to 6°C for use of the mixture within approximately 16 hours, or for storage at a temperature between 10 to 25°C for use of the mixture within approximately 4 hours,
ii. to a lyophilization device for lyophilization.

13. Method according to one of claims 2 to 12, **characterized in that** the homogenous mixture of fecal microbiota contains 15 bacterial genera *Blautia, Faecalibacterium, Alistipes, Eubacterium, Bifidobacterium, Ruminococcus, Clostridium, Coprococcus, Odoribacter, Roseburia, Holdemanella, Anaerostipes, Oscillibacter, Subdoligranulum* and *Butyrivibrio.*

14. Homogenous mixture of fecal microbiota capable of being obtained according to one of claims 1 to 13, for use thereof in the treatment of intestinal dysbioses and/or intestinal dysbioses associated diseases by transplantation of allogenic fecal microbiota.

15. Homogenous mixture of fecal microbiota capable of being obtained according to one of claims 1 to 13, **characterized in that** the mixture has an Inverse Simpson index greater than 15, preferably 20.

16. Homogenous mixture of fecal microbiota capable of being obtained according to one of claims 1 to 13, for use thereof in the treatment of intestinal dysbioses.

17. Homogenous mixture of fecal microbiota capable of being obtained according to one of claims 1 to 13, for use thereof in the treatment of graft-versus-host disease (GvHD).

18. Homogenous mixture of fecal microbiota capable of being obtained according to one of claims 1 to 13, for use thereof in the treatment of iatrogenic intestinal dysbiosis and/or associated pathologies and complications comprising intestinal inflammation, diarrhea.

19. Homogenous mixture of fecal microbiota capable of being obtained according to one of claims 1 to 13, for use thereof in the treatment of *Clostridium difficile* infection and associated diarrhea (CDI), inflammatory bowel disease (IBD), irritable bowel syndrome (IBS), idiopathic constipation, coeliac disease, Crohn's disease, obesity and morbid obesity, autism, multiple sclerosis, traveler's diarrhea, Parkinson's disease, type II diabetes, food allergies, cancer, Alzheimer's disease, schizophrenia and bipolar disorders, intestinal dysbiosis associated with antineoplastic chemotherapy or immunotherapy and alcohol- or non-alcohol-related liver disease.
